(19) Europäisches Patentamt
European Patent Office
Office européen des brevets

(11) **EP 4 664 464 A2**

(12) **EUROPEAN PATENT APPLICATION**

(43) Date of publication:
**17.12.2025 Bulletin 2025/51**

(21) Application number: **25201262.0**

(22) Date of filing: **09.09.2024**

(51) International Patent Classification (IPC):
***G16C 20/30*** (2019.01)

(52) Cooperative Patent Classification (CPC):
**G16C 20/30;** G16C 20/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.09.2023 US 202363537458 P**

(62) Document number(s) of the earlier application(s) in accordance with Art. 76 EPC:
**24199341.9 / 4 521 412**

(71) Applicant: **Tempus AI, Inc.**
**Chicago, IL 60654 (US)**

(72) Inventors:
• **Lonini, Luca**
  **Chicago, IL 60611 (US)**
• **Ho, Chi-Sing**
  **Redwood City, CA 94063 (US)**
• **Kannan, Madhavi**
  **Bloomington, IL 61704 (US)**
• **Schau, Geoffrey**
  **Portland, OR 97209 (US)**
• **Larsen, Brian**
  **Chicago, IL 60646 (US)**
• **Salahudeen, Ameen**
  **Oak Park, IL 60302 (US)**

(74) Representative: **Hofmann, Matthias**
**Boehmert & Boehmert**
**Anwaltspartnerschaft mbB**
**Pettenkoferstraße 22**
**80336 München (DE)**

Remarks:
This application was filed on 09.09.2025 as a divisional application to the application mentioned under INID code 62.

(54) **PREDICTING UNOBSERVED QUANTITATIVE MEASURES USING MACHINE LEARNING**

(57) A method, computing system, and computer-readable medium may receive and process de novo quantitative measures input using a trained machine learning model to generate one or more predicted experimental cell quantitative measures. A method, computing system, and computer-readable medium may receive training quantitative measures data; train a machine learning model to generate predicted experimental cell viability measures based on a de novo quantitative measures input, by processing the training quantitative measures data; and store the machine learning model as a trained machine learning model in a memory of a computer.

FIG. 1

**Description**

**FIELD**

The present disclosure is directed to methods and systems for predicting unobserved quantitative measures using machine learning, and more particularly, to techniques for processing *de novo* inputs using trained machine learning models to generate predicted experimental cellular quantitative measures.

**BACKGROUND**

The background description provided herein is for the purpose of generally presenting the context of the disclosure. Work of the presently named inventors, to the extent it is described in this background section, as well as aspects of the description that may not otherwise qualify as prior art at the time of filing, are neither expressly nor impliedly admitted as prior art against the present disclosure.

Measuring experimental characteristics (e.g., dose-response relationships for drugs) is difficult. Such relationships are often non-linear, meaning that the relationship between drug dose and its effect is not proportional. In some cases, low doses of a drug may have minimal effect, while a slight increase in dose can lead to a significant response. This non-linearity can complicate the assessment of synergistic effects, as the combined effect of two drugs may not follow a simple additive pattern.

Some drugs may reach a point of saturation where increasing the dose beyond a certain level does not produce a proportional increase in effect. This saturation effect can obscure the determination of synergistic interactions, as the response may plateau even when combining drugs with different doses.

Certain drugs may exhibit threshold effects, where their effects are not observed until a specific dose is reached. Below this threshold, the drug might have little or no effect. When combining drugs, determining the appropriate doses to achieve synergistic effects may be challenging, especially if the threshold doses for individual drugs differ significantly.

Obtaining complete dose-response data for individual drugs may be a key to accurately assessing synergistic interactions. However, in practice, researchers may only have access to limited data points due to logistical constraints or ethical considerations. This limited data can make it challenging to identify precise dose combinations that lead to synergistic effects.

The toxicity of drugs can also be dose-dependent. While lower doses may be well-tolerated, higher doses or drug combinations might lead to adverse reactions or harmful side effects. Balancing the desired therapeutic effects with potential toxicities is critical when investigating synergistic effects.

Some drugs may exhibit time-dependent effects, where their impact increases or decreases over time. This temporal aspect adds another layer of complexity to measuring synergistic effects, as the timing and sequence of drug administration can influence the overall response.

People can vary significantly in their responses to drug doses due to genetic differences, age, underlying health conditions, and other factors. The individual variability in dose-response relationships further complicates the assessment of synergistic effects in a diverse population.

Combination therapies, where two or more drugs are delivered simultaneously to treat a variety of medical conditions including cancer, can increase efficacy while simultaneously reducing individual drug doses, dosing intervals, and side-effects. To identify drugs that may have synergistic effects when combined, drug combinations are measured across a range of doses in preclinical in vitro or in vivo experiments at varying dose levels or concentrations. These results yield a dose-response matrix, which records the drug response/effect at the varied ratios of combination.

However, it is resource intensive to generate a dose-response matrix, compared to evaluating dose and response for a single agent, due to the exponential nature of evaluating two or more drugs in varying concentrations (e.g., an x-squared proposition for two drug experiments, an x-cubed proposition for three drug experiments, etc.; with x being the number of doses to test).

Overall, the dose-response characteristics of drugs play a significant role in understanding and measuring synergistic effects. The non-linearity, saturation, threshold effects, incomplete data, toxicity concerns, time-dependent effects, and individual variability all contribute to the complexities researchers encounter when studying drug combinations and their synergistic interactions.

Conventional techniques for predicting dose-response data are deficient, lacking the ability to calculate viability for missing doses, lacking the ability to calculate values for unmeasured time steps, lacking the ability to process image data and lacking the ability to augment training and operation data with additional input features.

Accordingly, there is an opportunity for improved and sophisticated quantitative measures prediction platforms and technologies.

## SUMMARY

In an aspect, a computer-implemented method for using a machine learning model trained using data to perform prediction of unmeasured experimental cell quantitative measures to reduce laboratory experimentation burden includes (i) receiving, via one or more processors, *de novo* input; and (ii) processing the *de novo* input using the machine learning model to generate one or more predicted experimental cell quantitative measures.

In some possible implementations of the first aspect, the machine learning model may be a regression machine learning model or a classification machine learning model. The *de novo* quantitative measures input may include a sparse quantitative measures matrix.

In some possible implementations of the first aspect, the regression machine learning model may be at least one of (i) a random forest model, (ii) an XGBoost model, (iii) an artificial neural network, (iv) a Gaussian regression model, (v) a support vector machine or (vi) a ridge and LASSO regression model.

In some possible implementations of the first aspect, the regression machine learning model may include a single or multi-target mean-squared-error cost function.

In some possible implementations of the first aspect, the predicted experimental cell quantitative measures may include at least one microscopy image associated with an experiment or experimental results.

In some possible implementations of the first aspect, the machine learning model may be trained on at least some masked training quantitative measures data.

In some possible implementations of the first aspect, the machine learning model may be an artificial neural network capable of encoding and decoding input images and trained using quantitative measures data including one or more brightfield training images and/or one or more fluorescent training images. And the *de novo* quantitative measures input may correspond to at least one point in a continuous latent space embedding.

In some possible implementations of the first aspect. the artificial neural network may be a variational autoencoder network, a transformer network, a recurrent neural network or a long short-term network.

In some possible implementations of the first aspect, the machine learning model may include: an encoder trained to map one or more portions of the brightfield or fluorescent training images to the continuous latent space embedding representing a compressed low dimensional representation of the one or more training images; and a decoder trained to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input.

In some possible implementations of the first aspect, the method may further include predicting viability or synergy scores or other quantitative measures metrics from images using the machine learning model.

In some possible implementations of the first aspect, the method may further include augmenting the continuous latent space embedding, using the encoder, by processing one or more human-interpretable morphological features.

In some possible implementations of the first aspect, the human-interpretable morphological features may be generated by a computer-vision technique.

In some possible implementations of the first aspect, the method may further include generating one or both of (i) a predicted viability corresponding to the de novo quantitative measures input, and (ii) a synergy map or score corresponding to the de novo quantitative measures input.

In some possible implementations of the first aspect, the predicted cell quantitative measures may correspond to at least one of missing time points, missing dose responses or missing immune ratios.

In a second aspect, a computer-implemented method for training a machine learning model to perform prediction of quantitative measures to identify compounds showing synergistic effects and reduce laboratory experimentation burden includes (i) receiving, via one or more processors, training quantitative measures data; (ii) training, via one or more processors, a machine learning model to generate predicted experimental cell viability measures based on a *de novo* quantitative measures input, by processing the training quantitative measures data; and (iii) storing, via one or more processors, the machine learning model as a trained machine learning model in a memory of a computer.

In some possible implementations of the second aspect, the machine learning model maybe a regression machine learning model. The training quantitative measures data may include one or more substantially dense or fully dense training quantitative measures matrices, each including respective experimental cell viability measures. The *de novo* quantitative measures input may include a sparse quantitative measures matrix.

In some possible implementations of the second aspect, the regression machine learning model maybe at least one of (i) a random forest model, (ii) an XGBoost model, (iii) an artificial neural network, (iv) a Gaussian regression model, (v) a support vector machine or (vi) a ridge and LASSO regression model.

In some possible implementations of the second aspect, the regression machine learning model may include a single or multi-target mean-squared-error cost function.

In some possible implementations of the second aspect, training the machine learning model may include:

receiving, via one or more processors, one or more additional input features; and processing the additional input features along with the one or more substantially dense or fully dense training quantitative measures matrices.

In some possible implementations of the second aspect, the additional input features may include at least one of (i) a fluorescent nuclear and chromosome counterstain value, (ii) an indication of cell death, (iii) an indication of cell apoptosis, (iv) a cancer type, (v) one or more image embeddings, (vi) one or more morphological features, (vii) one or more compound doses, (viii) one or more features encoding a molecular structure of a compound (ix) RNA sequencing data of cells, or (x) DNA sequencing data of cells.

In some possible implementations of the second aspect, training the machine learning model may include masking at least some of the training quantitative measures data.

In some possible implementations of the second aspect, the machine learning model may be a variational autoencoder. the training quantitative measures data may include one or more brightfield and/or fluorescent training images. And the *de novo* quantitative measures input may correspond to at least one point in a continuous latent space embedding.

In some implementations of the second aspect, training the machine learning model may include: training, via one or more processors, an encoder to map one or more portions of the training images to the continuous latent space embedding representing a compressed low dimensional representation of the one or more training images; and training, via one or more processors, a decoder to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input.

In some implementations of the second aspect, training the machine learning model may include: training an imaging-based regression model to predict viability or synergy scores from reconstructed images or from the continuous latent space embeddings.

In some implementations of the second aspect, training the imaging-based regression model may include training a fully-connected network to generate decoded viability based on inputs from a decoder.

In some implementations of the second aspect, the method may further include receiving human-interpretable morphological features, including at least one of size, shape, circularity, fractal dimension, texture and pixel intensity.

In some implementations of the second aspect, the human-interpretable morphological features may be generated by a computer-vision technique.

In some implementations of the second aspect, training the encoder may include augmenting the latent space embedding by processing one or more human-interpretable morphological features.

In some implementations of the second aspect, the method may further include training a regression head to generate one or both of (i) a predicted viability corresponding to the *de novo* quantitative measures input, and (ii) a synergy map or score corresponding to the *de novo* quantitative measures input.

In some implementations of the second aspect, training the machine learning model may include optimizing the regression head and the variational autoencoder by combining two loss functions.

In a third aspect, a computing system includes one or more processors; and one or more memories having stored thereon computer-executable instructions that, when executed by the one or more processors, cause the computing system to: (i) receive, via one or more processors, *de novo* quantitative measures input; and (ii) process the *de novo* quantitative measures input using the machine learning model to generate one or more predicted experimental cell quantitative measures.

In a fourth aspect, a computing system includes one or more processors; and one or more memories having stored thereon computer-executable instructions that, when executed by the one or more processors, cause the computing system to: (i) receive, via the one or more processors, training quantitative measures data; (ii) train, via the one or more processors, a machine learning model to generate predicted experimental cell viability measures based on a *de novo* quantitative measures input, by processing the training quantitative measures data; and (iii) store, via one or more processors, the machine learning model as a trained machine learning model in a memory.

In a fifth aspect, a computer-readable medium includes computer-executable instructions that, when executed by one or more processors, cause a computer to: (i) receive, via one or more processors, *de novo* quantitative measures input; and (ii) process the *de novo* quantitative measures input using the machine learning model to generate one or more predicted experimental cell quantitative measures.

In a sixth aspect, a computer-readable medium includes computer-executable instructions that, when executed by one or more processors, cause a computer to: (i) receive, via the one or more processors, training quantitative measures data; (ii) train, via the one or more processors, a machine learning model to generate predicted experimental cell viability measures based on a *de novo* quantitative measures input, by processing the training quantitative measures data; and (iii) store, via one or more processors, the machine learning model as a trained machine learning model in a memory.

Aspects of the techniques described in the present disclosure may include any of the following aspects, either alone or in combination:

1. A computer-implemented method for using a machine learning model trained using quantitative measures data to perform prediction of unmeasured quantitative measures experimental cell quantitative measures to reduce laboratory experimentation burden, the method comprising: receiving, via one or more processors, *de novo* quantitative measures input; and processing the *de novo* quantitative measures input using the machine learning model to generate one or more predicted experimental cell quantitative measures.

2. The computer-implemented method of aspect 1, wherein the machine learning model is a regression machine learning model or a classification machine learning model, wherein the *de novo* quantitative measures input includes a sparse quantitative measures matrix.

3. The computer-implemented method of any one of aspects 1-2, wherein the regression machine learning model is at least one of (i) a random forest model, (ii) an XGBoost model, (iii) an artificial neural network, (iv) a Gaussian regression model, (v) a support vector machine or (vi) a ridge and LASSO regression model.

4. The computer-implemented method of any one of aspects 1-3, wherein the regression machine learning model includes a single or multi-target mean-squared-error cost function.

5. The computer-implemented method of aspect 1, wherein the predicted experimental cell quantitative measures include at least one microscopy image associated with an experiment or experimental results.

6. The computer-implemented method of any one of aspects 1-2, wherein the machine learning model is trained on at least some masked training quantitative measures data.

7. The computer-implemented method of aspect 1, wherein the machine learning model is an artificial neural network capable of encoding and decoding input images and trained using quantitative measures data including one or more brightfield training images and/or one or more fluorescent training images; and wherein the *de novo* quantitative measures input corresponds to at least one point in a continuous latent space embedding.

8. The computer-implemented method of aspect 7, wherein the artificial neural network is a variational autoencoder network, a transformer network, a recurrent neural network or a long short-term network.

9. The computer-implemented method of any one of aspects 1-7, wherein the machine learning model includes: an encoder trained to map one or more portions of the brightfield or fluorescent training images to the continuous latent space embedding representing a compressed low dimensional representation of the one or more training images; and a decoder trained to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input.

10. The computer-implemented method of any one of aspects 1-9, further comprising: predicting viability or synergy scores or other quantitative measures metrics from images using the machine learning model.

11. The computer-implemented method of any one of aspects 1-9, further comprising: augmenting the continuous latent space embedding, using the encoder, by processing one or more human-interpretable morphological features.

12. The computer-implemented method of aspect 11, wherein the human-interpretable morphological features are generated by a computer-vision technique.

13. The computer-implemented method of any one of aspects 1-10, further comprising: generating one or both of (i) a predicted viability corresponding to the *de novo* quantitative measures input, and (ii) a synergy map or score corresponding to the *de novo* quantitative measures input.

14. The computer-implemented method of claim 1, wherein the predicted cell quantitative measures correspond to at least one of missing time points, missing dose responses or missing immune ratios.

15. A computer-implemented method for training a machine learning model to perform prediction of unmeasured quantitative measures to identify compounds showing synergistic effects and reduce laboratory experimentation burden, the method comprising: receiving, via one or more processors, training quantitative measures data; training, via one or more processors, a machine learning model to generate predicted experimental cell viability measures based on a *de novo* quantitative measures input, by processing the training quantitative measures data; and storing, via one or more processors, the machine learning model as a trained machine learning model in a memory of a computer.

16. The computer-implemented method of aspect 15, wherein the machine learning model is a regression machine learning model, wherein the training quantitative measures data includes one or more substantially dense or fully dense training quantitative measures matrices, each including respective experimental cell viability measures; and wherein the *de novo* quantitative measures input includes a sparse quantitative measures matrix.

17. The computer-implemented method of aspect 16, wherein the regression machine learning model is at least one of (i) a random forest model, (ii) an XGBoost model, (iii) an artificial neural network, (iv) a Gaussian regression model, (v) a support vector machine or (vi) a ridge and LASSO regression model.

18. The computer-implemented method of any of aspects 15-16, wherein the regression machine learning model includes a single or multi-target mean-squared-error cost function.

19. The computer-implemented method of any one of aspects 14-17, wherein training the machine learning model includes: receiving, via one or more processors, one or more additional input features; and processing the additional input features along with the one or more substantially dense or fully dense training quantitative measures matrices.

20. The computer-implemented method of aspect 19, wherein the additional input features include at least one of (i) a fluorescent nuclear and chromosome counterstain value, (ii) an indication of cell death, (iii) an indication of cell apoptosis, (iv) a cancer type, (v) one or more image embeddings, (vi) one or more morphological features, (vii) one or more compound doses, (viii) one or more features encoding a molecular structure of a compound (ix) RNA sequencing data of cells, or (x) DNA sequencing data of cells.

21. The computer-implemented method of any one of aspects 14-19,
wherein training the machine learning model includes masking at least some of the training quantitative measures data.

22. The computer-implemented method of any of aspects 14-20, wherein the machine learning model is a variational autoencoder, wherein the training quantitative measures data includes one or more brightfield and/or fluorescent training images; and wherein the *de novo* quantitative measures input corresponds to at least one point in a continuous latent space embedding.

23. The computer-implemented method of aspect 22, wherein training the machine learning model includes: training, via one or more processors, an encoder to map one or more portions of the training images to the continuous latent space embedding representing a compressed low dimensional representation of the one or more training images; and training, via one or more processors, a decoder to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input.

24. The computer-implemented method of any one of aspects 21-23, wherein training the machine learning model includes: training an imaging-based regression model to predict viability or synergy scores from reconstructed images or from the continuous latent space embeddings.

25. The computer-implemented method of any one of aspects 21-24, wherein training the imaging-based regression model includes training a fully-connected network to generate decoded viability based on inputs from a decoder.

26. The computer-implemented method of any one of aspects 14-24, further comprising: receiving human-interpretable morphological features, including at least one of size, shape, circularity, fractal dimension, texture and pixel intensity.

27. The computer-implemented method of aspect 26, wherein the human-interpretable morphological features are generated by a computer-vision technique.

28. The computer-implemented method of any one of aspects 21-25, wherein training the encoder includes augmenting the latent space embedding by processing one or more human-interpretable morphological features.

29. The computer-implemented method of any one of aspects 14-24, further comprising: training a regression head to generate one or both of (i) a predicted viability corresponding to the *de novo* quantitative measures input, and (ii) a synergy map or score corresponding to the *de novo* quantitative measures input.

30. The computer-implemented method of aspect 29, wherein training the machine learning model includes optimizing the regression head and the variational autoencoder by combining two loss functions.

31. A computing system, comprising: one or more processors; and one or more memories having stored thereon computer-executable instructions that, when executed by the one or more processors, cause the computing system to: receive, via one or more processors, *de novo* quantitative measures input; and process the *de novo* quantitative measures input using a machine learning model to generate one or more predicted experimental cell quantitative measures.

32. A computing system, comprising: one or more processors; and one or more memories having stored thereon computer-executable instructions that, when executed by the one or more processors, cause the computing system to: receive, via the one or more processors, training quantitative measures data; train, via the one or more processors, a machine learning model to generate predicted experimental cell viability measures based on a *de novo* quantitative measures input, by processing the training quantitative measures data; and store, via one or more processors, the machine learning model as a trained machine learning model in a memory.

33. A computer-readable medium having stored thereon computer-executable instructions that, when executed by one or more processors, cause a computer to: receive, via one or more processors, *de novo* quantitative measures input; and process the *de novo* quantitative measures input using a machine learning model to generate one or more predicted experimental cell quantitative measures.

34. A computer-readable medium having stored thereon computer-executable instructions that, when executed by one or more processors, cause a computer to: receive, via the one or more processors, training quantitative measures data; train, via the one or more processors, a machine learning model to generate predicted experimental cell viability measures based on a *de novo* quantitative measures input, by processing the training quantitative measures data; and store, via one or more processors, the machine learning model as a trained machine learning model in a memory.

## BRIEF DESCRIPTION OF THE DRAWINGS

The figures described below depict various aspects of the system and methods disclosed herein. It should be understood that each figure depicts an example of aspects of the present systems and methods.

FIG. 1 depicts an exemplary computing system for training and/or operating machine learning models to predict unmeasured quantitative measures using one or more trained machine learning models, according to some aspects.

FIG. 2 depicts a block flow diagram for using a trained machine learning model to predict complete or substantially complete dose-response quantitative measures, provided incomplete *de novo* quantitative measures, and for further processing the complete or substantially complete dose-response quantitative measures to generate a synergy distribution and/or synergy scores to identify dose pairs having synergistic effects, according to some aspects.

FIG. 3 depicts a block flow diagram for using a trained regression machine learning model to predict complete or substantially complete dose-response quantitative measures, provided incomplete *de novo* quantitative measures, according to some aspects.

FIG. 4A depicts an exemplary synergy score calculation with respect to a synergy score map, according to some aspects.

FIG. 4B depicts a three-dimensional synergy score map, according to some aspects.

FIG. 5A depicts an exemplary variational autoencoder architecture for predicting complete or substantially complete dose-response quantitative measures, according to some aspects.

FIG. 5B depicts a conceptual diagram of interpolating in latent space using the trained autoencoder architecture of FIG. 5A, according to some aspects.

FIG. 5C depicts an exemplary sparse dose-response matrix, according to some aspects.

FIG. 5D depicts an alternative autoencoder architecture, according to some aspects.

FIG. 5E depicts yet another alternative autoencoder architecture, according to some aspects.

FIG. 6 depicts a graph of exemplary experimental savings that may be realized by using the present techniques, according to some aspects.

FIG. 7A depicts a computer-implemented for using a machine learning model trained using dose-response data to perform prediction of unmeasured dose-response experimental cell quantitative measures to reduce laboratory experimentation burden, according to some aspects.

FIG. 7B depicts a computer-implemented method for training a machine learning model to perform prediction of unmeasured quantitative measures to identify compounds showing synergistic effects and reduce laboratory experimentation burden.

## DETAILED DESCRIPTION

### Overview

The present techniques are directed to methods and systems for predicting unmeasured quantitative measures using machine learning, and more particularly, to techniques for processing *de novo* quantitative measures inputs using trained machine learning models to generate predicted experimental cell quantitative measures. The present techniques may include using one or more machine learning model(s) to advantageously reduce the number of experiments needed to identify compounds having additive or synergistic effects.

Herein, the term "synergistic" compounds or substances are those that, when used together, exhibit a cooperative interaction resulting in a combined effect that is greater than the sum of their individual effects. The synergy between these compounds may enhance therapeutic outcomes, providing a more potent, beneficial, or desired response compared to using the compounds separately. Synergistic interactions may be positive or negative. For example, synergistic effects may include increased toxicity or adverse effects when combined. Thus, when exploring synergistic compounds, potential risks and benefits associated with their combined use must be carefully evaluated. In some aspects, the present techniques may identify antagonistic combinations to eliminate problematic combination regimens in drug development.

Herein, the "effect" in "synergistic effect" generally refers to the combined and enhanced ability of two or more therapeutic agents, treatments, or drugs to work together in a cooperative manner to more effectively destroy or inhibit the growth of tumor cells (for example, in a multi-cellular structure like a tumor or tumor organoid) compared to the individual effects of each agent. The present techniques may be configured to measure effects for organoids, including three-dimensional patient-derived organoids, in addition to, or alternative to, individual tumor cell(s), as discussed in more detail below.

Specifically, a "synergistic effect" in the context of killing tumor cells means that the combined action of the therapeutic agents or treatments results in a more significant reduction in tumor cell viability, proliferation, or survival than what would be achieved by using each agent separately. The combined effect is greater than the sum of the individual effects, providing a more potent and targeted approach to combating the tumor. Synergistic interactions in tumor cell killing can involve different mechanisms, such as increasing cell death (apoptosis), disrupting cell division and growth, inhibiting angiogenesis (formation of blood vessels to support tumor growth), enhancing immune response against the tumor, or a combination of these effects.

The present techniques may include techniques for training one or more machine learning model(s) to predict a full dose-response matrix (or tensor for more than two drugs) from a partial dose-response matrix data set that corresponds to a limited number of experiments. In addition, the present techniques may be extended to predict time-based quantitative measures, such as data for unmeasured time points, in the case of experiments measuring compound effects over time. This advantageously allows accelerating the rate of drug discovery by upscaling experimental resolution (for example, using empirical data to simulate additional data), thereby being able to test several more drug combinations in a preclinical research program. Specifically, the invention increases the throughput of modeling lab drug screening assays, allowing more experiments to be run using the same resources (in other words, generating the same size dataset of results using fewer experiments), thereby reducing costs and increasing environmental sustainability. Further, this advantageously prevents removing experimental plates from an incubator to image them, which affect how organoid cells grow, leading to data degradation and inaccurate experimental conclusions. In the present techniques, imputing data from intermediate timepoints enables the cells to remain undisturbed in incubators during the unmeasured intermediate time points, such that their growth is not altered by removal from an incubator.

These improvements in turn accelerate drug discovery while, at the same time, benefiting patients by allowing testing of more drug combinations to facilitate the deployment and delivery of personalized therapy.

In some aspects, the present techniques may be offered to another party, for example via a platform-as-a-service (PaaS) offering, a white labeled service, etc. Such other parties may include pharmaceutical companies, biotechnical companies, contract research organizations, startups (e.g., personalized medicine firms), healthcare analytics companies, software companies, pharmacogenomics companies, healthcare consultancies, non-profit research organizations, etc.

**Exemplary Computing Environments**

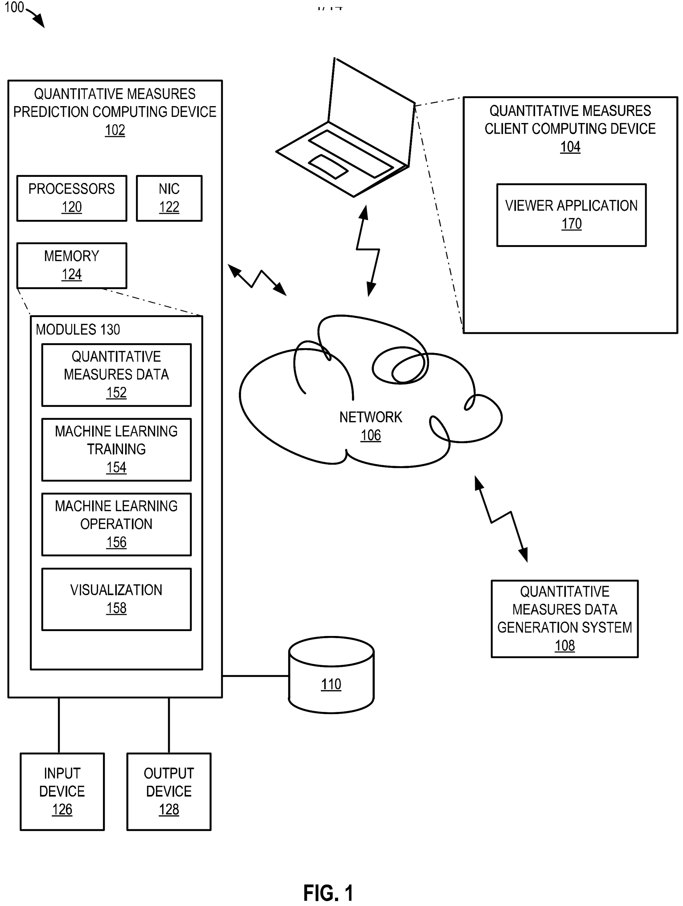

FIG. 1 illustrates an exemplary computing environment 100 for performing the present techniques, according to some aspects. The environment 100 may include computing resources for training and/or operating machine learning models to predict unmeasured quantitative measures using one or more trained machine learning models to process training and inference inputs, and for further processing/ computations of such predictions.

The computing environment 100 may include a quantitative measures prediction computing device 102, a quantitative measures client computing device 104, an electronic network 106, a quantitative measures data generation system 108 and an electronic database 110. The components of the computing environment 100 may be communicatively connected to one another via the electronic network 106, in some aspects.

The quantitative measures prediction computing device 102 may implement, *inter alia,* quantitative measures data receipt/storage/preprocessing, quantitative measures model training, quantitative measures model operation and related operations. In some aspects, the quantitative measures prediction computing device 102 may be implemented as one or more computing devices (e.g., one or more servers, one or more laptops, one or more mobile computing devices, one or more tablets, one or more wearable devices, one or more cloud-computing virtual instances, etc.). The quantitative measures analysis computing device 102 may include one or more processors 120, one or more network interface controllers 122, one or more memories 124, an input device 126 and an output device 128.

In some aspects, the one or more processors 120 may include one or more central processing units, one or more graphics processing units, one or more field-programmable gate arrays, one or more application-specific integrated circuits, one or more tensor processing units, one or more digital signal processors, one or more neural processing units, one or more RISC-V processors, one or more coprocessors, one or more specialized processors/accelerators for artificial intelligence or machine learning-specific applications, one or more microcontrollers, etc.

The quantitative measures prediction computing device 102 may include one or more network interface controllers 122, such as Ethernet network interface controllers, wireless network interface controllers, etc. The network interface controllers 122 may include advanced features, in some aspects, such as hardware acceleration, specialized networking protocols, etc.

The memories 124 of the quantitative measures prediction computing device 102 may include volatile and/or non-volatile storage media. For example, the memories 124 may include one or more random access memories, one or more read-only memories, one or more cache memories, one or more hard disk drives, one or more solid-state drives, one or more non-volatile memory express, one or more optical drives, one or more universal serial bus flash drives, one or more external hard drives, one or more network-attached storage devices, one or more cloud storage instances, one or more tape drives, etc.

As noted, the memories 124 may have stored thereon the modules 130, for example, as one or more sets of computer-executable instructions. In some aspects, the modules 130 may include additional storage, such as one or more operating systems (e.g., Microsoft Windows, GNU/Linux, Mac OSX, etc.). The operating systems may be configured to run the modules 130 during operation of the quantitative measures prediction computing system 102 - for example, the modules 130 may include additional modules and/or services for receiving and processing quantitative measures data. The modules 130 may be implemented using any suitable computer programming language(s) (e.g., Python, JavaScript, C, C++, Rust, C#, Swift, Java, Go, LISP, Ruby, Fortran, etc.).

The modules 130 may include a quantitative measures data module 152, a machine learning training module 154, a machine learning operation module 156 and a visualization module 158. In some aspects, more or fewer modules 130 may be included. The modules 130 may be configured to communicate with one other (e.g., via inter-process communication, via a bus, etc.).

The quantitative measures data module 152 may include instructions for receiving, retrieving, pre-processing and/or storing quantitative measures data. Herein, quantitative measures data may be incomplete or complete quantitative measures. For example, the quantitative measures data module 152 may include instructions for receiving incomplete quantitative measures, as discussed herein (e.g., via the input device 128, as discussed below). Generally, the quantitative measures may relate to drug dosage, immune/non-immune cell ratio, time (for example, treatment time or time spent in a certain condition), etc. The quantitative measures data may include one or more types of data of non-image modality, such as tabular data, text input, binary streams, etc. The instructions may include instructions for preprocessing the quantitative measures or other quantitative measures data (e.g., metadata). In some aspects, the quantitative measures data module 152 may include instructions for accessing (e.g., storing and/or retrieving) the quantitative measures data.

For example, the quantitative measures data module 152 may access the quantitative measures data via the memory 124 (e.g., in a flat file, a compressed file, etc.) and/or via the electronic database 110 (e.g., via an insert, update, upsert, select query). Generally, the quantitative measures data module 152 may make data available for processing by other elements of the computing environment 100 (e.g., the other modules 130). In some aspects, the quantitative measures data module 152 may preprocess the quantitative measures data.

For example, in some aspects, the input device 126 may be an image capture device. The quantitative measures data module 152 may receive medical images, for example including microscopy images, histopathology slides such as digital hematoxylin and eosin (H&E stained slide images, immunohistochemistry (IHC) stained slide images, and/or digital images of any other staining protocol(s) from any suitable source(s).

In some aspects, the quantitative measures data module 152 may receive physician clinical records and/or histopathology imaging data, for example from the electronic database 110 or via the input device 126 or via another computing environment accessible via the network 106. Any number of medical image data sources may be accessible using the computing environment 100. The histopathology images may include one or more images captured by any dedicated digital medical image scanner(s), e.g., any suitable optical histopathology slide scanner including magnified (e.g., 20x and 40x resolution) scanners. Further still, quantitative measures data module 152 may receive images from one or more histopathology image repositories (e.g., one or more electronic databases, non-transitory computer-readable memories, etc.). In yet other examples, images may be received from a partner genomic sequencing system, e.g., the TCGA and NCI Genomic Data Commons. Further still, the quantitative measures analysis computing device may receive histopathology images from an organoid modeling lab.

The quantitative measures data module 152 may receive image data, genomic data, patient data, treatment data, historical data, etc., in accordance with the techniques and processes described herein. The quantitative measures data module 152 may receive image data from multiple image sources, in some aspects. The multiple image sources may be different, and capable of generating and providing imaging data that differs from other providers, hospitals, etc. The quantitative measures data module 152 may receive imaging data from different sources that differ in one or more ways, resulting in different data source-specific bias, such as in different dyes, biospecimen fixations, embeddings, staining protocols, and distinct pathology imaging instruments and settings.

The quantitative measures data module 152 may perform pre-processing to enhance image data for faster processing (e.g., for training a machine learning framework). For example, the quantitative measures data module 152 may perform a normalization process on received image data, including one or more of color normalization, intensity normalization, imaging source normalization, etc. The quantitative measures data module 152 may perform the normalization to compensate for and correct for differences in received image data. While in some examples the quantitative measures data module 152 receives medical images, in other examples the sub-system is able to generate medical images (e.g., via the input device 126 and/or output device 128).

The quantitative measures data module 152 may perform further image processing that removes artifacts and other noise from received images by doing preliminary tissue detection, for example, to identify regions of the images corresponding to histopathology stained tissue for subsequent analysis, classification, and segmentation.

In some aspects, such as where image data is to be analyzed on a tile-basis, the quantitative measures data module 152 may pre-process images by receiving an initial histopathology image, at a first image resolution, downsampling that image to a second image resolution, and then performing a normalization on the downsampled histopathology image, such as color and/or intensity normalization, and removing non-tissue objects from the image.

**Exemplary Computer-Implemented Machine Learning Model Training Aspects**

The machine learning training module 154 may include instructions for training one or more machine learning models, according to the techniques discussed herein. For example, in some aspects, the machine learning module 154 may include one or more sets of instructions for implementing various machine learning techniques to perform quantitative measures analysis based on information such as the quantitative measures data received by the quantitative measures data module 152.

For example, in some aspects, the machine learning training module 154 may include instructions for training one or more generative models (e.g., a variational autoencoder machine learning model, regression and classification models, etc.). The machine learning training module 154 may train such a model to predict complete or substantially complete quantitative measures (e.g., unobserved/unmeasured/missing/corrupted dose response measures, imputed time points, immune cell activity, etc.), as discussed in more detail herein. The unobserved quantitative measures may be unobserved due to choice, faulty equipment, experimental design, etc. Generally, the machine learning training module 154 may include instructions for training one or more variational autoencoder models by mapping inputs (e.g., images) to a continuous latent space, wherein the variational autoencoder learns to recreate the inputs via sampling of the latent space. This trained model may then be used in practice (e.g., via the machine learning operation module 156) to predict quantitative measures at runtime based on *de novo* input data (e.g., data accessible via the quantitative measures data module 152) including an input incomplete matrix or tensor of values. Herein "de novo" data refers to data that a model has not seen before (e.g., data that was held out while training the model, or data made accessible to the model after the model was trained). The incomplete data may correspond to experimental measurements (e.g., a sampling of dose-dose response data). Thus, the machine learning training module 154 may train models trained to automatically generate experimental results, *in silico,* without having to actually perform those results. Avoiding exhaustive experimentation is a particularly advantageous and compelling improvement provided to automated quantitative measures prediction systems, provided by the present techniques.

As discussed above, many types of training data may be used in conjunction with the present machine learning models. For example, the machine learning training module 154 may train the one or more machine learning models using training quantitative measures including drug efficacy, viability, cell proliferation rate, apoptosis rate (cells in the process of dying), cell migration/invasion rate, gene expression levels, enzyme activity, cell cycle analysis, reactive oxygen species, cytokine/chemokine release, metabolic activity, neuronal activity, cell morphology, cell adhesion, binding affinity, etc. In some aspects, the machine learning training module 154 may train one or more model using numeric quantitative measures. In some aspects, the machine learning training module 154 may train one or more models using training quantitative measures that are expressed using other data modalities, such as microscopy image inputs, text inputs, tabular inputs, etc. Herein, data modalities may be distinct from data representations/substance. For example, a microscopy image may be provided directly to a model as an input, or it may be encoded into a different format (e.g., a binary format, a text-based format, etc.). In still further aspects, the training quantitative measures may correspond to measures of off-target, general cell toxicity/cytotoxicity (e.g., also killing healthy non-cancer cells) or adverse effects.

The machine learning training module 154 may train one or more models using image data that has been pre-processed as discussed above, images from H&E slides or images from IHC slides (with or without human annotation), including IHC slides targeting PD-L1, PTEN, EGFR, Beta catenin/catenin beta1, NTRK, HRD, PIK3CA, and hormone receptors including HER2, AR, ER, and PR. In some aspects, training data may include DNA sequences, RNA sequences, metabolomics data, proteomic/cytokine data, epigenomic data, organoid data, raw karyotype data, transcription data, transcriptomics, metabolomics, microbiomics, and immunomics, identification of SNP, MNP, InDel, MSI, TMB, CNV Fusions, loss of heterozygosity, loss or gain of function. Epigenomic data includes DNA methylation, histone modification, or other factors which deactivate a gene or cause alterations to gene function without altering the sequence of nucleotides in the gene. Microbiomics includes data on viral infections which may affect treatment and diagnosis of certain illnesses as well as the bacteria present in the patient's gastrointestinal tract which may affect the efficacy of medicines ingested by the patient. Proteomic data includes protein composition, structure, and activity; when and where proteins are expressed; rates of protein production, degradation, and steady-state abundance; how proteins are modified, for example, post-translational modifications such as phosphorylation; the movement of proteins between subcellular compartments; the involvement of proteins in metabolic pathways; how proteins interact with one another; or modifications to the protein after translation from the RNA such as phosphorylation, ubiquitination, methylation, acetylation, glycosylation, oxidation, or nitrosylation.

Training data may further include demographic data and tumor response data (including data about a reduction in the growth of the tumor after exposure to certain therapies, for example immunotherapies, DNA damaging therapies like PARP inhibitors or platinums, or HDAC inhibitors). The demographic data may include age, gender, race, national origin, etc. The tumor response data may include epigenomic data, examples of which include alterations in chromatin morphology and histone modifications.

The tumor response data may include cellular pathways, examples of which include IFN gamma, EGFR, MAP KINASE, mTOR, CYP, CIMP, and AKT pathways, as well as pathways downstream of HER2 and other hormone receptors. The tumor response data may include cell state indicators, examples of which include Collagen composition, appearance, or refractivity (for example, extracellular vs fibroblast, nodular fasciitis), density of stroma or other stromal characteristics (for example, thickness of stroma, wet vs. dry) and/or angiogenesis or general appearance of vasculature (including distribution of vasculature in collagen/stroma, also described as epithelial-mesenchymal transition or EMT). The tumor response data may include tumor characteristics, examples of which include the presence of tumor budding or other morphological features/characteristics demonstrating tumor complexity, tumor size (including the bulky or light status of a tumor), aggressiveness of tumor (for example, known as high grade basaloid tumor, especially in colorectal cancer, or high grade dysplasia, especially in Barrett's esophagus), and/or the immune state of a tumor (for example, inflamed/"hot" vs. non-inflamed/"cold" vs immune excluded). The trained models module 136 may load training data from the database 110, for example.

The machine learning training module 154 may be configured to train any number of machine learning models using any suitable machine learning techniques, including, for example, a variational autoencoder, a regression model, a fully-connected network, deep learning techniques, probabilistic encoders/decoders, embedding vectors, an artificial neural network, a convolutional neural network, a recurrent neural network, etc. The machine learning training module 154 may store one or more trained model, including model weights/hyperparameters and/or other model metadata in a suitable location for later retrieval, such as the memory 124 and/or in the electronic database 110.

With trained classifier models, the machine learning framework may be used to analyze and diagnose quantitative measures analysis in subsequent images collected from patients over time. In this manner, images and other data of previously treated and analyzed patients is utilized, through the trained models, to provide analysis and diagnosis capabilities for future patients.

The quantitative measures data module 152 may be a standalone system interfacing with external (e.g., third party) network-accessible systems. In some examples, the quantitative measures data module 152 may be integrated with one or more of these systems, including as part of a distributed cloud-based platform. Any of the functions described in the techniques herein may be distributed across one or more network accessible devices, including cloud-based devices.

In some examples, the quantitative measures data module 152 may be part of a comprehensive quantitative measures analysis prediction, diagnosis and treatment system. For example, the quantitative measures data module 152 quantitative measures analysis computing device 104 may be configured to communicate quantitative measures analysis computing information to external systems, including a computer-based pathology lab/oncology system that may receive generated quantitative measures information and use the same for further diagnosing cancer state of the patient and for identifying matching therapies for use in treating the patient. The quantitative measures analysis computing device 104 may further send generated reports to a computer system of the patient's primary care provider and/or to a physician clinical records system for storing the patient's report with previously generated reports on the patient and/or with databases of generated reports on other patients for use in future patient analyses, including machine learning-based analyses, such as those described herein.

**Exemplary Computer-Implemented Machine Learning Model Operation**

The machine learning operation module 156 may operate one or more models trained by the machine learning training module 154, in some aspects. Once trained, the machine learning operation module 156 may load one or more trained models (e.g., from the database 110). The machine learning operation module 156 may include instructions for preparing the loaded model for use, such as installing dependencies, downloading one or more pre-trained models, downloading the model itself, loading one or more model architectures (e.g., having one or more layers, connections and/or parameters), loading the pre-trained weights into corresponding layers of the model, and performing one or more inference/prediction sub-operations, such as performing forward inference. In some aspects, the machine learning operation module 156 may be configured to perform post-processing and/or fine-tuning based on model outputs.

The machine learning operation module 156 and/or another component may load the trained model (e.g., by accessing the trained models database 110). In some aspects, trained models may be stored and accessed via a memory of the computing device 102, and/or via a distributed memory (e.g., via the network 106).

The quantitative measures data module 152 may be a standalone system interfacing with external (e.g., third party) network-accessible systems. In some examples, the quantitative measures data module 152 may be integrated with one or more of these systems, including as part of a distributed cloud-based platform. For example, the quantitative measures data module 152 may be integrated with a histopathology imaging system, such as a digital H&E stain imaging system, e.g. to allow for expedited quantitative measures analysis and reporting at an imaging station. Indeed, any of the functions described in the techniques herein may be distributed across one or more network accessible devices, including cloud-based devices. Histopathology slides may be used to confirm that an organoid is a certain cancer type, or is not healthy tissue, for example.

In some examples, the quantitative measures data module 152 may be part of a comprehensive quantitative measures analysis prediction, diagnosis and treatment system. For example, the quantitative measures data module 152 quantitative measures of analysis computing device 104 may be configured to communicate quantitative measures analysis computing information to external systems, including a computer-based pathology lab/oncology system that may receive generated quantitative measures information and use the same for further diagnosing cancer state of the patient and for identifying matching therapies for use in treating the patient. The quantitative measures analysis computing device 104 may further send generated reports to a computer system of the patient's primary care provider and/or to a physician clinical records system for storing the patient's report with previously generated reports on the patient and/or with databases of generated reports on other patients for use in future patient analyses, including machine learning-based analyses, such as those described herein.

The quantitative measures client computing device 104 may be configured to access quantitative measures data, and to receive results (e.g., results generated by the machine learning operation module 156). For example, the quantitative measures client computing device 104 may include one or more processors, memories and/or network interface controllers (not depicted) that have similar functionality as those of the quantitative measures prediction computing device 102. The memory of the quantitative measures client computing device 104 may include one or more sets of instructions that perform functions that are different from those of the quantitative measures prediction computing device 102. For example, the quantitative measures client computing device 104 may include a viewer application 170 that includes instructions for accessing and displaying results generated by the machine learning operation module 156. Thus, the viewer application 170 may enable a user (e.g., an employee, a contractor, a third-party, a physician, a diagnostician, a software engineer, a system administrator, etc.) to review results generated via the present techniques.

In some aspects, the viewer application 170 may include instructions that allow the user to operate the one or more trained models operated by the machine learning operation module 156 in different ways (e.g., by parameterizing their inputs differently) and/or to modify the training parameters of one or more networks trained by the machine learning training module 154. The viewer application 170 may include instructions that allow the user to download results, share results (e.g., via email), and/or to visualize results (e.g., via causing one or more charts or graphs to be displayed via the quantitative measures client computing device 104). The viewer application 170 may include instructions that allow the viewer application 170 to access the components of the computing environment 100 (e.g., the quantitative measures prediction computing device 102, the electronic network 110, etc.) via the network 106, and components for displaying visualizations generated by the quantitative measures prediction computing device 102.

**Exemplary Visualization Generation Aspects**

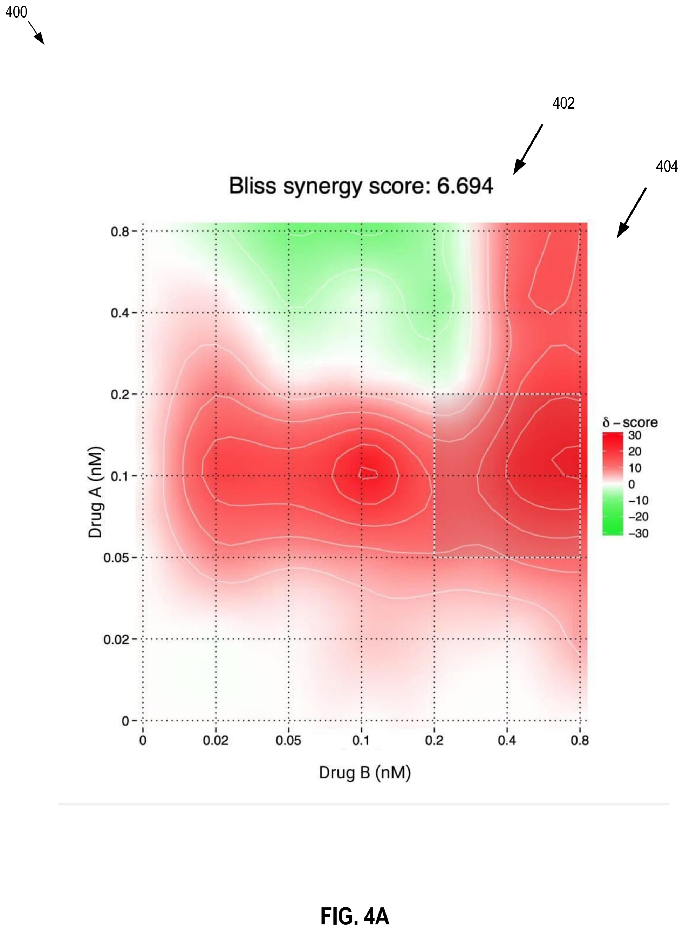

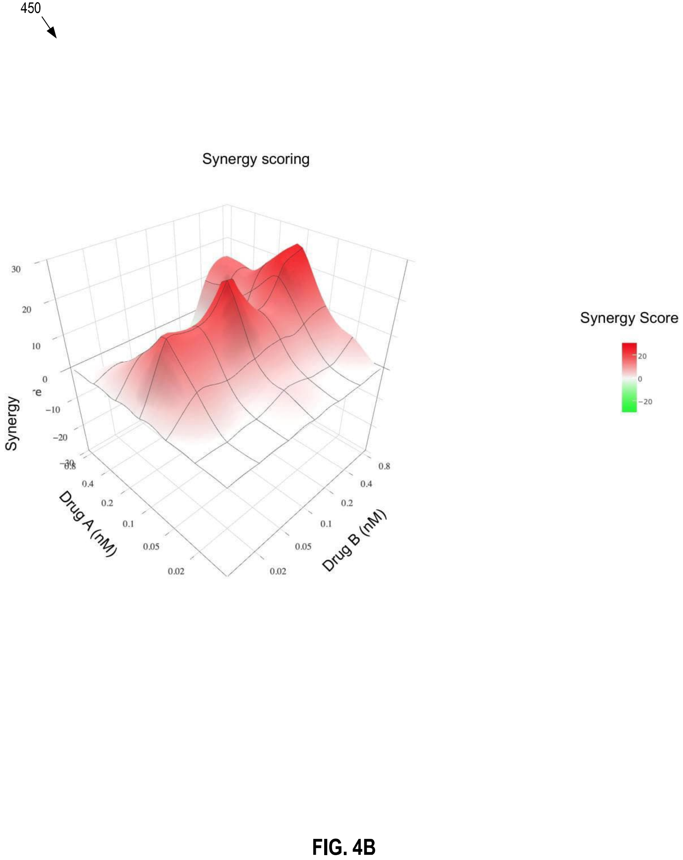

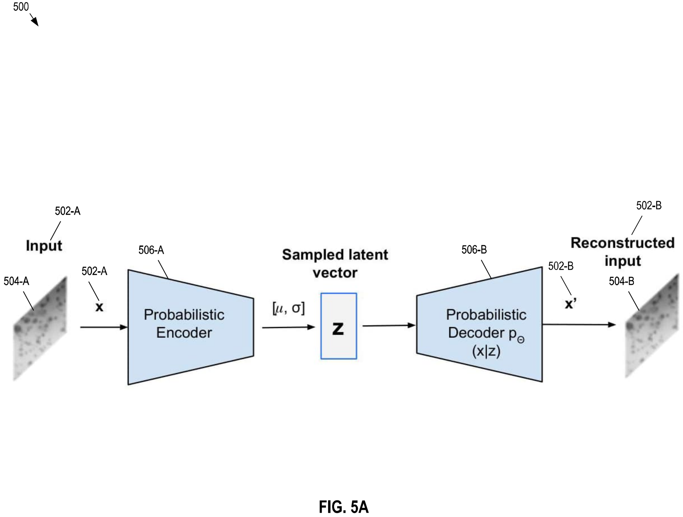

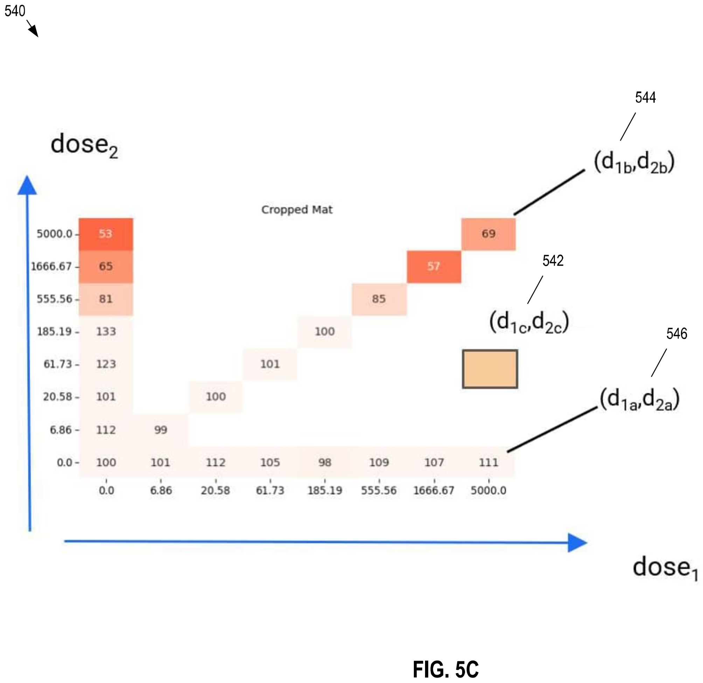

The visualization module 158 may include sets of computer-executable instructions for generating visualizations of quantitative measures data and outputs generated by training one or more machine learning models using the machine learning training module 154 and/or operating one or more machine learning models using the machine learning operation module 156. For example, the visualization module 158 may include a set of computer-executable instructions for generating drug-drug synergy score calculations and related synergy score maps, according to some aspects. For example, the visualization module 158 may generate a synergy score map like that of FIG. 4A, in some aspects. In some aspects, the visualization module 158 may generate a three-dimensional synergy score map like that of FIG. 4B. In some aspects, the visualization module 158 may generate an incomplete matrix visualization like that of FIG. 5C, or a complete matrix visualization.

The visualization module 158 may cause the generated visualization(s) to be stored (e.g., in the memory 124 or the electronic database 110), and/or to be displayed directly (e.g., via the output device 128, which may be or include a display device in some aspects), and/or to be transmitted via the network 106 (e.g., for display via the viewer application 170).

The electronic network 106 may communicatively couple the elements of the environment 100. The network 106 may include public network(s) such as the Internet, a private network such as a research institution or corporation private network, and/or any combination thereof. The network 106 may include a local area network (LAN), a wide area network (WAN), a cellular network, a satellite network, and/or other network infrastructure, whether wireless or wired. In some aspects, the network 106 may be communicatively coupled to and/or part of a cloud-based platform (e.g., a cloud computing infrastructure). The network 106 may utilize communications protocols, including packet-based and/or datagram-based protocols such as Internet protocol, transmission control protocol, user datagram protocol, and/or other types of protocols. The network 106 may include one or more devices that facilitate network communications and/or form a hardware basis for the networks, such as one or more switches, one or more routers, one or more gateways, one or more access points (such as a wireless access point), one or more firewalls, one or more base stations, one or more repeaters, one or more backbone devices, etc.

The quantitative measures generation system 108 may include any computing system, machine or software that generates quantitative measures data that may be used to train the machine learning models as discussed herein and/or for predictive/classification purposes using the trained machine learning models discussed herein. For example, the quantitative measures data generation system 108 may generate the quantitative measures data received by the quantitative measures data module 152 discussed above, such as a microscope or repository of digital microscopy images, a histopathology slide generator or repository thereof, an immunohistochemistry slide image generator or repository thereof, or any source of digital data (including images, text data, tabular data, binary data, etc.).

The electronic database 110 may include one or more suitable electronic databases for storing and retrieving data, such as relational database (e.g., MySQL databases, Oracle databases, Microsoft SQL Server databases, PostgreSQL databases, etc.). The electronic database 110 may be a NoSQL database, such as a key-value store, a graph database, a document store, etc. The electronic database 110 may be an object-oriented database, a hierarchical database, a spatial database, a time-series database, an in-memory database, etc. In some aspects, some or all of the electronic database 110 may be distributed.

In some aspects, some or all of the components of the environment 100 may be implemented as a cloud computing instance (e.g., the electronic database 110 may be a cloud computing database instance, the quantitative measures prediction computing device 102 may be implemented as one or more cloud computing instances, etc.). Any suitable cloud computing infrastructure may be used to implement cloud-based components, including Amazon Web Services, Microsoft Azure, Google Cloud Platform, etc. Further, in some aspects, some of the components of the environment 100 may be implemented using a private cloud infrastructure and/or a hybrid cloud infrastructure.

In operation, a user may access the environment 100 (e.g., via the quantitative measures client computing environment 104) to configure one or more training data sets (e.g., via the quantitative measures data module 152) and one or more untrained models (e.g., via the machine learning training module 154). The user may cause the configuration of training data sets and untrained models to be performed by accessing elements of the viewer application 170 (e.g., via a graphical user interface). The user may cause a training procedure to be performed, by accessing the machine learning operation module 156.

In some aspects, the data preparation, model configuration and training processes may be performed automatically, for example, using a scheduler (e.g., Cron), a timer, a task scheduler, a PowerShell script, or another automation tool/framework (e.g., Ansible, Chef, Puppet, etc.). The user and/or automation tool may cause a training procedure to occur, followed by an operation (i.e., inference) operation once the training procedure concludes. The user may view the output of the inference, and share the results (e.g., with a third party via email). In some aspects, the quantitative measures prediction computing device 102 may include instructions for performing further processing of the results of the machine learning, such as generating visualizations as described above, via the visualization module 158.

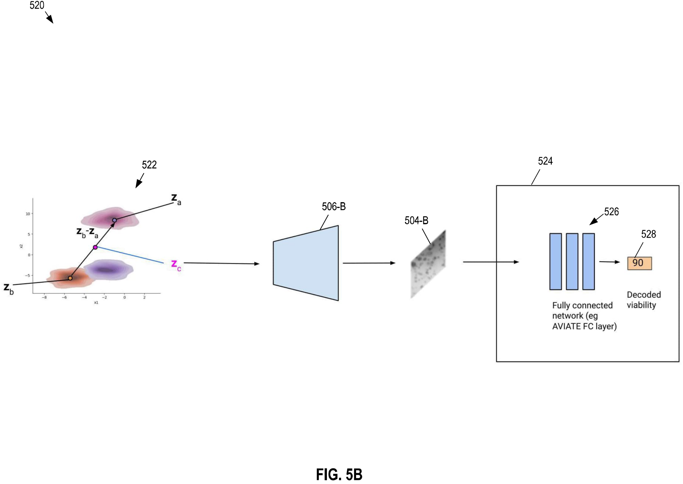

In some aspects, the user may use the environment 100 to configure training and/or operation of one or more autoencoder architectures, such as the autoencoder architecture 500 depicted in FIG. 5A, below. The user may further configure training and/or operation of one or more regressors, such as the regressor 524 of FIG. 5B. The user may select (e.g., via a graphical use interface displayed on the viewer application 170 of FIG. 1) a data set of incomplete quantitative measures and cause the environment 100 to train one or more machine learning models using that data set. The user may then select one or more *de novo* inputs (i.e., inputs that the model was not trained with) and provide them to the environment 100 to cause the trained model to process those *de novo* inputs, and to provide predictions based thereon. For example, the user may provide an incomplete matrix, such as the sparse dose-response matrix of FIG. 5C, at runtime for inference/prediction as a *de novo* input.

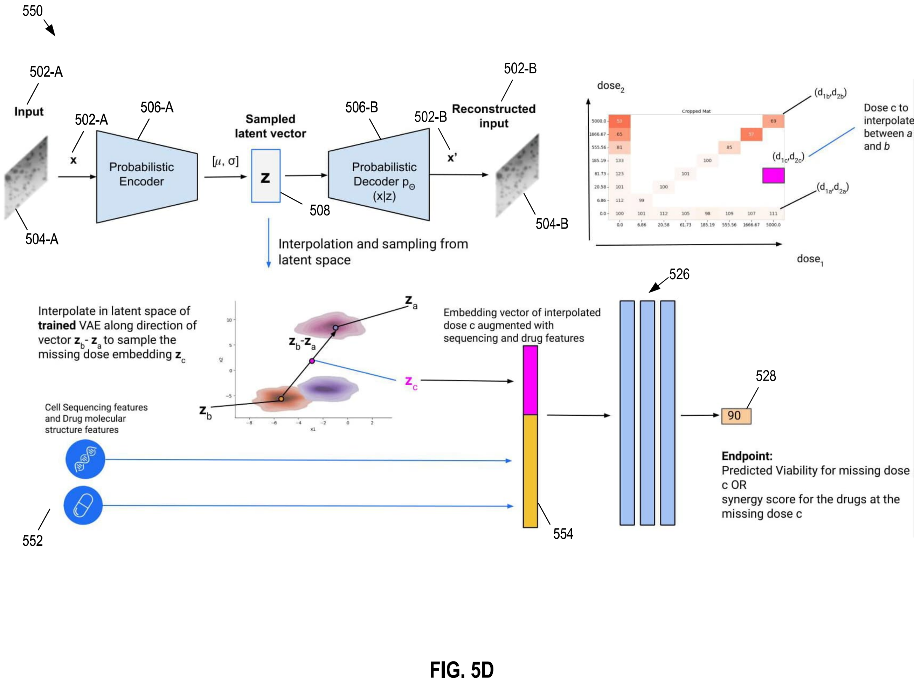

Further, more complex arrangements of models may be configured, trained and operated by the user (or automatically, as discussed above), such as the models of FIG. 5D, in which a decoder head 554 is parameterized using latent space interpolations and additional features, for enriched predictability. It should be appreciated that the ability of the present techniques to further enhance variational autoencoder predictions using additional features is an improvement over any conventional technique that may apply a generative approach, and leads to improved predictions.

**Exemplary Regression or Classification-Based Dose-Response Prediction**

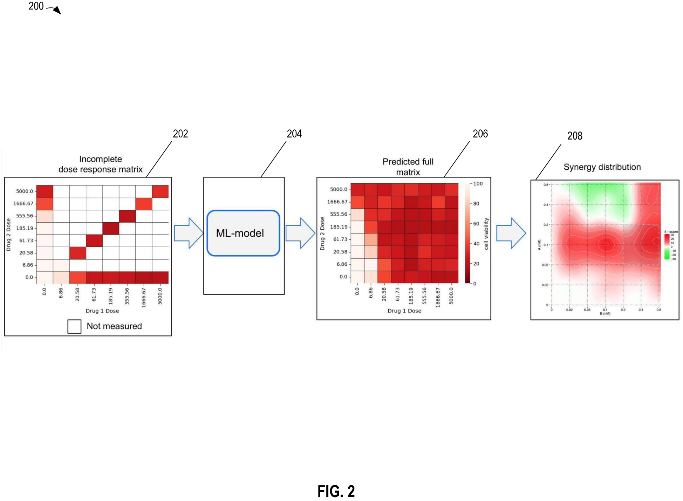

FIG. 2 depicts a block flow diagram 200 for using a trained machine learning model to predict complete or substantially complete dose-response quantitative measures, provided incomplete *de novo* quantitative measures, and for further processing the complete or substantially complete dose-response quantitative measures to generate a synergy distribution and/or synergy scores to identify dose pairs having synergistic effects, according to some aspects. Generally, the quantitative measures may include drug efficacy, viability, cell proliferation rate, apoptosis rate, cell migration/invasion rate, gene expression levels, enzyme activity, cell cycle analysis, reactive oxygen species, cytokine/chemokine release, metabolic activity, neuronal activity, cell morphology, cell adhesion, binding affinity, etc. In some aspects, the quantitative measures may be numeric and/or time-based. In other aspects, the measures may be expressed using other data modalities, such as microscopy image inputs, text inputs (e.g., SMILES-encoded data), tabular inputs, etc. In still further aspects, the quantitative measures may correspond to measures of off-target, general cell toxicity/cytotoxicity (e.g., also killing healthy non-cancer cells) or adverse effects.

The block flow diagram 200 includes receiving incomplete quantitative measures (depicted as a plurality of sparse matrices) (block 202). Herein, "sparse" may have its strict mathematical definition (e.g., that the number of zero elements is much higher than the number non-zero elements in a given matrix) or a more relaxed definition, wherein the matrix is technically incomplete, insofar as at least one element/entry of the matrix has a null, undefined or zero value. The quantitative measures may be expressed using any suitable data structure, and may include numeric information or other information (e.g., image-based information).

The block flow diagram 200 includes processing the quantitative measures using a trained machine learning model (block 204) to predict one or more quantitative measures, to complete the sparse matrices (block 206). In the depicted example, the machine learning model processes sparse matrices, wherein the sparsity of the matrices refers to unmeasured (i.e., incomplete, or intermediate) measurements for one or more drug-drug combinations. As depicted, the matrices represent a cartesian product of concentrations of two drugs (Drug 1, along the X axis; and Drug 2, along the Y axis) as measured in nanomoles. Of course, the drugs may be transposed (e.g., arranged in row-major or column-major order). The values of the matrices represent clinical result values, such as relative inhibition percentage. The values may also correspond to a percentage of total cells that are not dead or dying, referred to as "viability." In various embodiments, the tumor organoids may be grown with non-cancer cells, including immune cells, or any animal cell. For example, the present techniques may be used in conjunction with those described in U.S. Patent Application 17/816,395, entitled "Platform For Co-Culture Imaging To Characterize In Vitro Efficacy Of Heterotypic Effector Cellular Therapies In Cancer," filed on July 29, 2022, and hereby incorporated by reference in its entirety, for all purposes.

For example, with reference to USSN 17/816,395, methods for characterizing cancer organoid responses in response to an immune cell-based therapy may be extended using the present techniques. In an aspect, one or more incomplete quantitative measurements corresponding to the cancer organoid responses may be provided to the present techniques, which may process those measurements using the trained machine learning models discussed herein to generate complete/ substantially complete quantitative measures. In particular, the incomplete quantitative measures may correspond to measures corresponding to various dosing schedules of the cancer organoid and/or immune cells in conjunction with the cancer organoid, and responses thereof (e.g., morphology changes). For example, an organoid may be combined with immune cells in an experimental environment. The experimental design may determine the ratio of immune cells (effector cells) to organoid cells (target cells) (for example, 5:1, 4:1, 3:1, 2:1, 1:1, 1:2, 1:3, 1:4, 1:5, etc.). This environment may then be dosed with one or more drugs. The drug may not kill an organoid directly, but rather, increase immune cell response to cause cells to be killed. In some examples, the immune cells may be engineered to directly kill the organoid cells with or without the addition of drugs. Thus, organoid death may still be observed, and data related to sparse drug dosing may be collected and processed using the present techniques to predict data for intermediate doses and/or effector to target cell ratios that were not experimentally tested in the wet lab. In some aspects, data may measure the effect of immune cells on organoid cells, with no drug added. The present techniques may measure and predict different concentrations of and/or distances between immune and/or organoid/tissue cells.

Advantageously, this may enable the present techniques to significantly reduce the complexity (e.g., number, time, etc.) of experiments needed to characterize cancer organoid response. It should be appreciated that many other experimental techniques may be accelerated in this way.

Types of agents / drugs that may be used in dose-response experiments as discussed herein may include immune cell based therapeutic agents such as cellular therapeutics, as well as large molecule (biologics) and small molecule (pharmaceutical) agents that modulate immune cell activity. Examples of adoptive cell therapies include, for example, tumor-infiltrating lymphocyte therapy and immune cells (such as T cells and NK cells) engineered to express chimeric antigen receptor (CAR T-cells, CAR NK cells). Autologous or allogeneic effectors cells are contemplated, as well as autologous or allogeneic engineered or synthetic effector cell populations such as CAR T-cells. Examples of CAR T cell therapies include, but are not limited to, Axicabtagene ciloleucel, Brexucabtagene autoleucel, Idecabtagene vicleucel , Lisocabtagene maraleucel, and Tisagenlecleucel. Examples of biologics include recombinant proteins, such as antibodies and antibody-like constructs, which modulate the activity of immune cells and/or recruit immune effector cells to tumor cells. In various aspects, the immune cell based therapeutic is recombinant protein (e.g., an antibody or antibody-like construct) which engages/activates effector immune cells when in the presence of the tumor organoid cells. For example, in some aspects, the immune cell based therapeutic is a bispecific antibody or bispecific antibody-like construct (such as scFv-based constructs, bispecific Fabs, diabodies, and the like) which binds an immune effector cell and a tumor cell antigen. In various aspects, the antibody or antibody-like construct is a checkpoint inhibitor. The immune cell based therapeutic also may be DNA or RNA-based, such as RNA-based nanoparticles which upregulate T cell activation. Alternatively, the immune cell based therapeutic may be a small molecule drug, such as those which inhibit PI3K or Btk or activate T cells via, e.g., TLRs.

In various embodiments, the experiment may include exposing non-cancerous cells to a therapy and/or combinations of therapies, then measuring the viability of the cells. This will measure off-target effects of the therap(y/ies) (for example, to observe whether the cancer therapeutics will also negatively affect healthy, non-cancer cells).

Here, in general, the term "viability" refers to the ability of tumor cells to survive, grow, and proliferate. It is a measure of how healthy and functional the tumor cells are and indicates their potential to continue dividing and forming new cells. A decrease in cell viability indicates that the treatment is having a cytotoxic effect on the tumor cells, leading to reduced survival and growth. On the other hand, if cell viability remains high, it suggests that the treatment may not be effectively targeting the tumor cells or that the tumor is resistant to the treatment. In various embodiments, a treatment could have a cytostatic effect on the tumor cells, preventing the existing cells from dividing and increasing the total number of cells. In this case, a high percentage of the cells would be expected to remain viable but the total cell count would be lower than expected for the amount of time that the cells have been growing and/or significantly lower than a group of cells grown for a similar amount of time without the presence of the treatment. Thus, the present techniques may be configured to predict different aspects of viability. In some aspects, one or more models may be trained to quantify cytotoxic and/or cytostatic effects.

The present techniques may complete the sparse matrices by destructive modification of existing data structures (e.g., by inserting model-predicted values into the sparse matrix). Doing so may be more computationally efficient than creating copies of the sparse matrices. In any event, predicting the one or more quantitative measures and completing the matrices may result in one or more fully dense or substantially dense matrices corresponding to the incomplete/ sparse matrices, wherein "fully dense" means all elements/ entries in the completed matrices having predicted dose-dose values (e.g., relative inhibition percentage values) and "substantially dense" means nearly all elements/ entries in the completed matrices having predicted dose-dose values. Herein "dense matrix" refers to a fully dense or substantially dense matrix.

The block flow diagram 200 includes generating a synergy distribution map including a plurality of synergy scores (i.e., a synergy distribution) with respect to the dense matrices predicted at block 204 (block 208). In the depicted example, the drug-drug combinations in the central as well as upper right quadrant of the synergy distribution map indicates generally higher synergy scores, indicating drug-drug dose combinations that may have increased relative inhibition.

In general, one way that the present techniques significantly and advantageously improve the functioning of a computer, or another technology or technical field (e.g., computerized drug screening assays) is by reducing the number of experiments (e.g., testing intermediate doses) required to identify combinations of compounds that show synergistic effects. Further advantages are discussed in relation to the following diagrams.

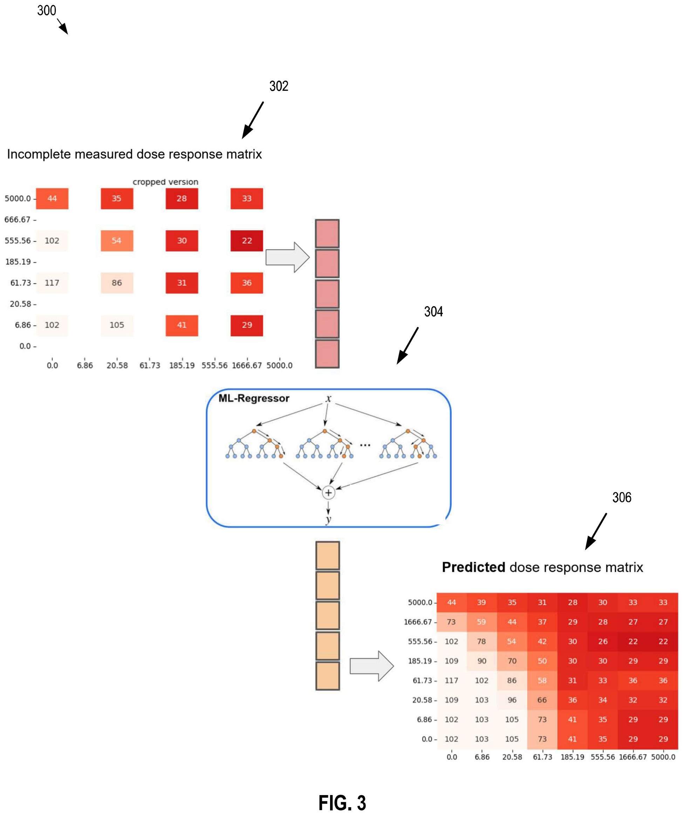

FIG. 3 depicts a block flow diagram 300 for using a trained regression machine learning model (block 304) to predict complete or substantially complete dose-response quantitative measures (block 306), by processing incomplete *de novo* quantitative measures (block 302). In general, the block flow diagram 300 may correspond to a technique including an *in vitro* assay, experimental and imaging protocol to quantify the effect of each compound and dose combination on cell death, using vital dye stains (e.g., TOPRO3 and Caspase 3/7) intensity measurements as endpoints, in some examples.

The machine learning model at block 304 may correspond to the machine learning model at block 204 of FIG. 2, in some aspects. The dose response matrix at block 302 is sparse or incomplete, insofar as it includes many intermediate, unmeasured values. The machine learning model at block 304 may be a regression model, for example, trained to interpolate viability for unmeasured doses from the dose-response matrix at block 302. The machine learning model may be any suitable model that performs regression or classification.

The machine learning model at block 304 may be a parametric or non-parametric machine learning regression or statistical model (e.g., ensembles of bagged and/or boosted trees, such as random forest or XGBoost, k-nearest-neighbor, artificial neural network, Support Vector Machines, Gaussian regression, polynomial regression, ridge regression, linear and LASSO regression, non-negative matrix factorization, etc.) that receives as input one or more of the following input quantitative measures features:

(1) cell viability for measured combination doses (e.g., mean and standard deviation and/or other statistical measures of TOPRO3 or Caspase 3/7 and/or other vital dye stains across cells) of two or more drugs;
(2) cancer type;
(3) dose combination values and, for multi-time points experiments (for example, where measurements are taken at multiple times after the initiation of treatment), time point (for example, duration of treatment at the time of measurement); and for experiments involving cell therapies, the ratio of effector to target cells;
(4) DNA/RNA sequencing data of cells / tumor organoids;
(5) image-derived (for example, visually detectable by human or computer) morphological (human-interpretable) features including cell size, shape, circularity, texture, pixel intensity, fractal dimension, etc., that can be computed: (i) at the image level (for example, per image, the calculations could done for an aggregate of two or more organoids in an image) using standard computer vision methods; (ii) at the organoid level (for example, per organoid, done for each organoid), using a segmentation technique that can segment the organoids; and/or (iii) morphological features at the organoid level calculated using computer vision after segmentation is performed;

(6) image embeddings obtained by passing unlabeled brightfield and/or fluorescence and/or cell painting images through a pre-trained deep learning autoencoder, convolutional neural network, transformer or any other deep learning architecture trained using unsupervised, or self-supervised learning; and/or

(7) drug molecular structure features (e.g., simplified molecular-input line-entry system or SMILES). The machine learning model at block 304 may impute intermediate time points (e.g., impute unmeasured data corresponding to points of time that fall between two times when data was actually measured).

The machine learning model at block 304 may output predicted cell viability for unmeasured dose combinations and/or time points and/or an effector to target cell ratio (e.g., in case of therapies involving immune cells). For example, activity may be measured at multiple different time points. In general, the present techniques may be used to predict missing time points within an experiment, in addition to (or as an alternative to) missing doses. Some experiments may have as parameters time points, immune dosages, and immune ratios. Any combination of these may be predicted using the present techniques. Morphological features may be cellular (e.g., relate to a biological cell or many biological cells/an organoid).

For example, the machine learning model at block 304 may be a random forest model, an XGBoost model, an artificial neural network, a Gaussian regression model, a support vector machine or a ridge and LASSO regression model. In some aspects, the machine learning model may be an ensemble of a plurality of different machine learning models (e.g., two or more regression models).

The machine learning model may be trained (e.g., by a training module stored in the memory 160 of FIG. 1B) by receiving and processing a training dataset of dose-response experiments (e.g., from the database 110) that includes one or more complete or substantially complete/ dense dose response matrices (or tensors, in case more than two compounds are used) for several experiments. This training dataset may further contain respective cell viability values for each measured dose. Next, the training module may apply a masking procedure such that a set of values from the dose-response matrix are removed or obscured. The masking procedure may include a modified training data set or a new training data set that is incomplete, where only a subset of dose responses (experiments) is measured. This masked data may be used as an input training dataset by the training module. The output (target) dataset used for training may include unmasked/ non-masked dose response matrices. The training module may train the machine learning model to predict these masked (i.e., unmeasured or missing) dose responses from the input set (and one or more other additional features, as discussed herein). The training procedure may be considered to be a supervised process, when the target values (e.g., cell viability) are known. The training module may train the machine learning model to predict more than one unmeasured dose at once (i.e., train a multi-output model) or to predict a single dose (e.g., train a single output model). Further, as discussed herein, the present techniques may include predicting viability (or a different output) at different points in time or different treatment variables, such as an effector to target cell ratio. In this case, the masked values may correspond to experiments at different time steps/points (or immune-tumor cell ratio), and the model may be trained to predict viability for an unmeasured time step (and optionally, a unmeasured dose or unmeasured immune-tumor cell ratio).

Once trained, the machine learning model at block 304 may receive input features such as measured mean cell viability (e.g., from TOPRO3 staining). The machine learning model may process the input features to generate model output, including unmeasured, predicted viability doses. The machine learning model may be configured to have a cost function (e.g., a single cost function, a multi-target mean-squared error function, etc.). In some aspects, the input features may include respective mean, an average and/or standard deviation of viability measures (e.g., TOPRO3, Caspase, etc.), one or more doses, one or more cancer types, one or more image embeddings, one or more morphological features, etc. In some aspects, the machine learning model at block 304 may be a single-output/ single dose regression model.

Predicting the dose response matrix at block 306 may include storing the predicted values in a non-transitory computer memory, such as the memory 160 of FIG. 1B, the database 110 of FIG. 1A, etc. Predicting the dose response may also include transmitting one or more predicted values and/or matrices via the communication network 106 of FIG. 1A and FIG. 1B, in some aspects.

**Exemplary Computer-Implemented Synergy Score and Summary Synergy Score Computations**

FIG. 4A depicts an exemplary synergy score calculation 402 with respect to a synergy score map 404.

In general, synergy scores measure the amount of combination synergy or antagonism between compounds (e.g., drugs) being tested. Synergy scores may be computed by comparing observed drug combination responses against expected responses, computed using a reference model that assumes no interaction between the drugs. Multiple synergy modeling algorithms may be used, including Bliss, Highest Single Agent (HSA), Loewe and/or Zero Interaction Potency (ZIP). Further, in some aspects, a plurality of these models may be combined to provide a consensus scoring, that may advantageously further reduce the risk of false positive synergy results, as described in Ianevski, Aleksandr, Anil K. Giri, and Tero Aittokallio. "SynergyFinder 3.0: an interactive analysis and consensus interpretation of multi-drug synergies across multiple samples." Nucleic acids research 50.W1 (2022): W739-W743, herein incorporated by reference in its entirety for all purposes.

For example, Bliss synergy assumes drugs elicit their effects independently. Bliss synergy is computed as:

$$Sbliss = EA,B(d1,d2) - (1 - (1 - EA(d1))(1 - EB(d2)))$$

where EA(d1) and EB(d2) are the individual effects of drug A and B at dose 1 and 2, respectively, and EA,B(d1,d2) is the combined effect measured experimentally. Here, effect may refer to cell inhibition, i.e. the proportion of cells killed by a compound. For example one may find that the effect for drug A is 30% (i.e. drug A kills 30% of cells) and for drug B is 60%. The combined effect EA,B(d1,d2) measured in the experiment is 80%. Bliss model expects the combination to kill 72% of cells (second term of the equation). The difference between the measured effect EA,B(d1,d2) and the expected effect is the synergy Sbliss = 0.08 (8%).

In some aspects, summary synergy scores may be computed as average excess responses across doses. For example:

Less than -10: the interaction between two drugs is likely to be antagonistic;
From -10 to 10: the interaction between two drugs is likely to be additive;
Larger than 10: the interaction between two drugs is likely to be synergistic.

In some aspects, open source packages may be used to compute synergy scores. One example is Synergy-Finder which is available for the R programming language; another is the Python package Synergy (Wooten, David J., and Réka Albert. "synergy: a Python library for calculating, analyzing and visualizing drug combination synergy." Bioinformatics 37.10 (2021): 1473-1474).

FIG. 4B depicts a three-dimensional synergy score map 450, according to some aspects.

**Exemplary Imaging-Based Generative Dose-Response Prediction**

The present techniques may include training a deep neural network encoding model (convolutional neural network, variational autoencoder, generative adversarial network, transformer, RNN and/or LSTM) trained on label-free images (e.g., brightfield and/or fluorescence and/or cell painting) to represent the images in a compressed (latent) space. Training may be performed using unsupervised or self-supervised learning, in some aspects. For example, training may be performed via the computing environment 100 of FIG. 1A.

FIG. 5A depicts an exemplary variational autoencoder architecture 500 for predicting complete or substantially complete dose-response quantitative measures. The variational autoencoder is configured to map one or more inputs 502-A (e.g., brightfield and/or fluorescence images 504-A) to a continuous latent space 508 from which samples can be taken, by training a probabilistic encoder 506-A and a probabilistic decoder 506-B of the autoencoder architecture 500 until one or more reconstructed inputs 502-B resemble the inputs 502-A.

Once the autoencoder architecture 500 is trained, the latent space 508 may be sampled to generate unmeasured dose images 504-B that include quantitative measures (e.g., viability of unmeasured doses).

FIG. 5B depicts a conceptual diagram 520 of interpolating in latent space 522 using the trained autoencoder architecture 500 of FIG. 5A. Interpolation in the latent space between doses may be performed to reconstruct the embedding of the image for the unmeasured dose and/or time point/step. The interpolation may include identifying extrema points in an embedding space and interpolating between them.

For example, as shown in FIG. 5B, the values of two points in latent space, $Z_a$ and $Z_b$, are known as they are part of the reduced-dimensionality representation of the input image 504-A. $Z_a$ may be a first extrema point that is a centroid of images treated with a maximum dosage of a drug, and $Z_b$ may be a second extrema point that is a centroid of images treated with a minimum dosage of a drug. A value of another point in latent space, $Z_c$, is not known, but its value can be interpolated by sampling the latent space along a direction of a vector $Z_b$-$Z_a$ using the trained probabilistic decoder 506-B to generate the unmeasured dose images 504-B. The vector connecting these two points estimates the dose-response curve. Sampling along this vector gives embeddings of intermediate interpolated samples. The embedding vector is decoded to generate the data point (image) corresponding to the unmeasured dose and/or time point. Decoding these samples with a generative model will produce images that resemble the raw images, which can provide explainability of the model. This approach is extensible to multiple extrema points, so multi-drug combination assessment is envisioned (e.g., from untreated to a first drug, from untreated to a second drug, and from the first drug to and from the second drug). In general, the latent space can be sampled along two (or more) directions of the hyperplane defined by three (or more) treatment centroids; this enables the generation of data for intermediate conditions defined by the treatment centroids. In other words, the present techniques may be used to perform synthetic experiments of drug combination, at respectively configurable dosages, by choosing appropriate points on the hyperplane/ latent space and decoding with the decoder 506-B.

The embedding vector may be augmented with a set of morphological human-interpretable features at image and/or organoid level, extracted from the input images (including size of cells, shape, texture and pixel intensity), in some aspects. As shown below (e.g., in FIG. 5D) the embedding vector may be augmented using modalities/data other than/in addition to image information, such as cell sequencing features, drug molecular structure features, etc.

As discussed below, a second neural network (e.g., an image-based classifier or a regression head) may input the generated image or its embedding vector and outputs: (1) the predicted cell viability for the unmeasured dose combination; (2) the predicted cell viability for an unmeasured time point; or (3) the predicted synergy map or synergy score of the compounds.

The sampled values may be passed to an image-based regressor 524. In some aspects, the regressor 524 may include a fully-connected network 526 capable of processing image embeddings, as discussed, for example, in: U.S. Patent Application No. 17/301,975, entitled "Artificial Fluorescent Image Systems and Methods," filed on April 20, 2021; U.S. Patent Application No. 17/857,901, entitled "Large Scale Organoid Analysis," filed on July 5, 2022; and U.S. Patent Application No. 17/114,386, entitled "Large Scale Organoid Analysis," filed on December 7, 2020; each of which is hereby incorporated by reference in its respective entirety, for all purposes. For example, hardware and software as discussed in USSN 17/301,975 may be used to automate image capture, such as brightfield microscopes, and the same with added optical accessories or capabilities (e.g., dark-field microscopy, phase-contrast microscopy, differential interference contrast microscopy, polarized light microscopy, confocal microscopy, light sheet microscopy, etc.). The present techniques may process brightfield images and/or fluorescent, both during and training and during inference. Raw brightfield images may be stored in the database 110, for example, along with respective three-channel brightfield images. The raw and/or three-channel brightfield images may be used for training one or more machine learning models. For example, the inputs 502-A and/or 504-A may include fluorescent and/or brightfield images, in raw and or channel. For example, the three channels of data in the three-channel brightfield (or fluorescent) images may include a blue/all nuclei channel, a green/apoptotic channel, and a red/pink/dead channel. Each channel can be used to form a fluorescent image. In some aspects, a machine learning model may be trained to output combined three-channel fluorescent images that include the blue/all nuclei channel, the green/apoptotic channel, and the red/pink/dead channel.

The regressor 524 may be trained to predict decoded information 528 (e.g., a viability, a synergy score, or other information) from images. It should be appreciated that it is possible to extend the method to interpolate across time points, for experiments with images acquired at multiple times.

In general, training of the autoencoder architecture 500 may be performed as follows: first, the autoencoder architecture 500 is trained on a dataset of brightfield and/or fluorescence images and/or cell painting images. These images may correspond to a variety of testing conditions, including different compound doses (in combination or alone), cancer lines, timepoints (for multi-timepoint experiments), and any other conditions that define the experiment. The autoencoder 500 is trained to only reconstruct the input image, so that the image reconstruction error is minimized; thus, the network does not require any labels associated with the input images at this stage, and this phase of training may be characterized as unsupervised. As noted, the autoencoder may also trained on other data modalities besides images, such as DNA/RNA cell sequencing data, drug molecular structure features and human interpretable image morphological features.

Once the autoencoder 500 is trained, a second network (e.g., the network 526, a regression head, etc.) may be trained to predict the endpoint (e.g., predicted viability for missed dose, synergy score, etc.) from the autoencoder image embeddings. The endpoint can be, for example, the viability, or the synergy score of the compounds, or a classification of whether the compounds are synergistic based on the current image embedding (i.e., the experiment corresponding to the input image). This phase of training may be supervised; as such, for each input embedding a label is provided (viability, synergy score or binary value on synergistic effects). The regression head may be trained using these labels to predict a synergy score, a viability score, etc. In general, the autoencoder 500 is trained in a label-free manner (i.e., unsupervised training), whereas the network 526 is trained using labels (i.e., supervised training).

At inference time, the present techniques may sample an embedding from the latent space, which corresponds to an intermediate dose or timepoint. The resulting sampled embedding is passed to the second network to predict the endpoint.

FIG. 5C depicts an exemplary sparse dose-response matrix 540. The sparse dose-response matrix 540 includes many intermediate/ incomplete values, such as the value 542 (coordinates: d1c,d2c). Values 544 (coordinates: d1b,d2b) and 546 (coordinates: d1a,d2a) are known, and may be used to predict, impute or interpolate the value 542 using any of the above-described techniques.

As described, the present techniques include machine learning / deep learning models to predict quantitative measures (e.g., cell viability, response to drug, etc.) for unmeasured dose-response measurements. It should be appreciated that variations on the above-described autoencoder architecture 500 are envisioned, as discussed with respect to the following figures.

FIG. 5D depicts an alternative autoencoder architecture 550, according to some aspects. In some aspects, the autoencoder architecture 550 may be identical to the architecture 500 of FIG. 5A in some/all respects, except that the autoencoder architecture 550 may include one or more additional input features 552 and one or more corresponding embeddings 554. The embeddings 554 may be referred to as an embedding vector. In some aspects, the additional input features 552 may represent additional raw input features (e.g., RNA/DNA sequencing data), and the corresponding embedding vector 554 may be a concatenation of the additional input features 552 (e.g., a feature vector) with the embeddings 554 (e.g., an embedding vector). Further, the regression head 526 may be trained to predict viability for the current input image from the image latent space embedding augmented with the additional feature embeddings 554. The embedding vector 554 may similarly be augmented with cell sequencing data (e.g. DNA/RNA) and/or drug molecular structure features (e.g. SMILES notation). In some aspects, the present techniques may include sampling an embedding vector with additional features (e.g., cell sequencing data such as RNA/DNA, drug molecular structure features for example as expressed in SMILES notation, etc.) In some aspects, the autoencoder architecture 550 may be trained on tabular data (e.g., morphological image features, cell sequencing data, drug molecular structure data) rather than on the images themselves. The output of the regression head may be either a viability or a synergy score map, indicating that the compounds are synergistic, additive, or antagonistic.

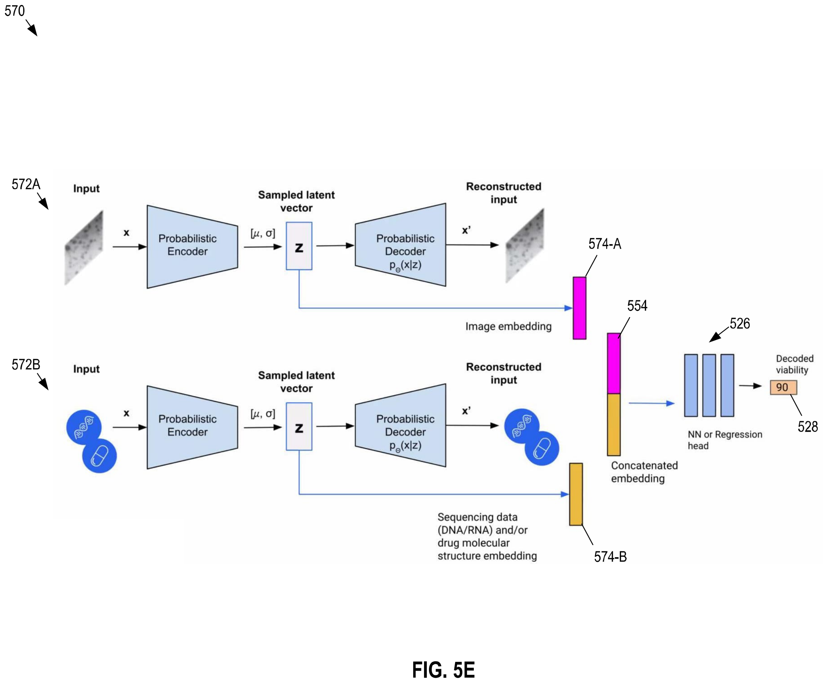

FIG. 5E depicts a further alternative autoencoder architecture 570, having a first autoencoder 572-A and a second autoencoder 572-B, according to some aspects of the present techniques. The autoencoder 572-A and the autoencoder 572-B may, respectively, generate image embeddings 574-A and feature embeddings 574-B. The embeddings 574 may be concatenated to generate the embeddings 554, that may correspond to the embeddings 554 of FIG. 5D. In FIG. 5E, the second autoencoder 572-B may encode input features using an architecture that is similar to the architecture of the autoencoder 572-A. The architecture 570 may sample embeddings from the autoencoders 572-A and 572-B and concatenate those embeddings to form the embeddings 554, which may be further processed by the regression head 526 to generate the decoded information 528.

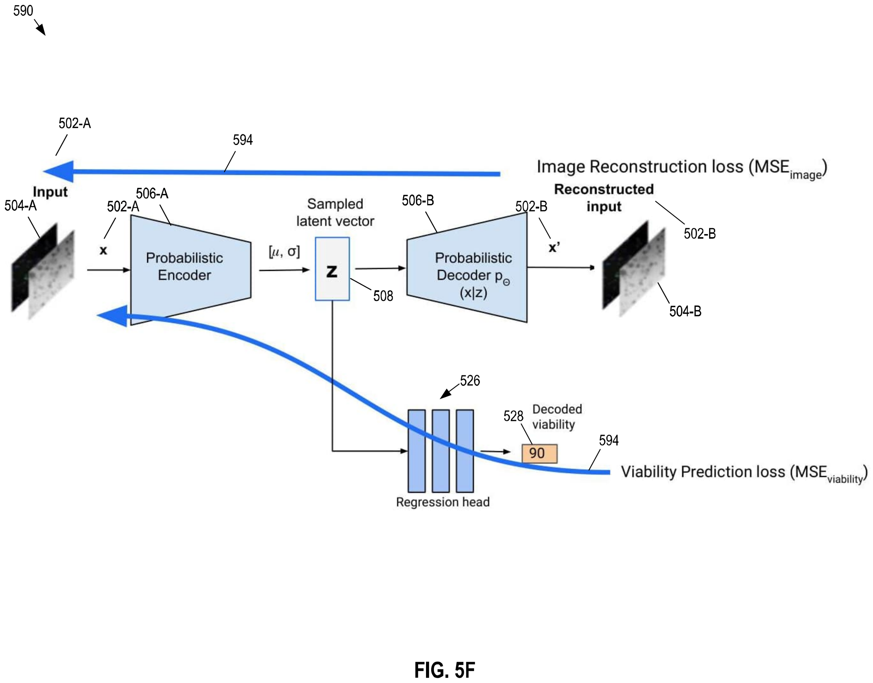

FIG. 5F depicts yet another alternative autoencoder architecture 590, according to some aspects. The autoencoder architecture 590 may differ from the autoencoder 500 of FIG. 5A and the autoencoder architecture 550 of FIG. 5D, for example, by including structure to jointly optimize the regression head 526 with the autoencoder 590 by combining two loss functions (image reconstruction loss, or $MSE_{image}$ and viability prediction loss $MSE_{viability}$). The architecture 590 is expected to generate a latent space 508 where close points represent images with similar viability values. The arrows 594 indicate that the loss values are backpropagated through the decoder head and regression head, respectively.

Returning to FIG. 1B, in some aspects, the machine learning operation module 154, for example, may include a set of computer-executable instructions that, when executed, cause a computer (e.g., the computing device 104) to select one or more trained model (e.g., the regression model 304 of FIG. 3, the autoencoder 500 of FIG. 5A, etc.) based on inputs provided to the controller. For example, the instructions may retrieve a model from the database 110 based on the input(s) provided to the machine learning operation module 154.

## Exemplary Advantages

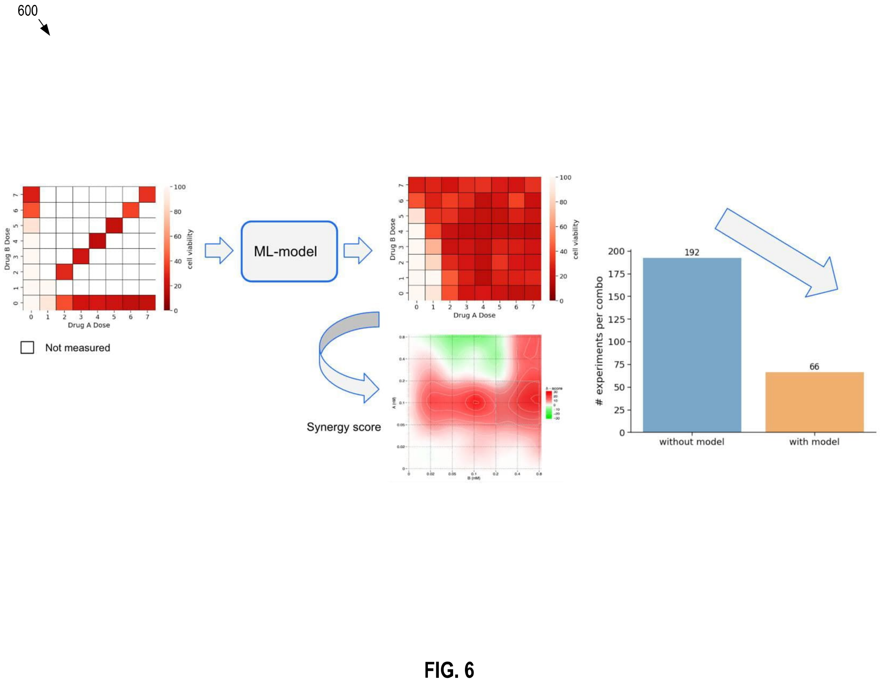

The present techniques advantageously reduce the size of experiments (as measured by reagents, plate wells, cells/organoids, etc.) required to be performed for a given modeling task. For example, FIG. 6 depicts a graph 600 of exemplary experimental savings that may be realized by using the present techniques. Empirical testing has demonstrated a reduction in the number of required experiments by as much as 66%, or more.

## Exemplary Computer-Implemented Methods

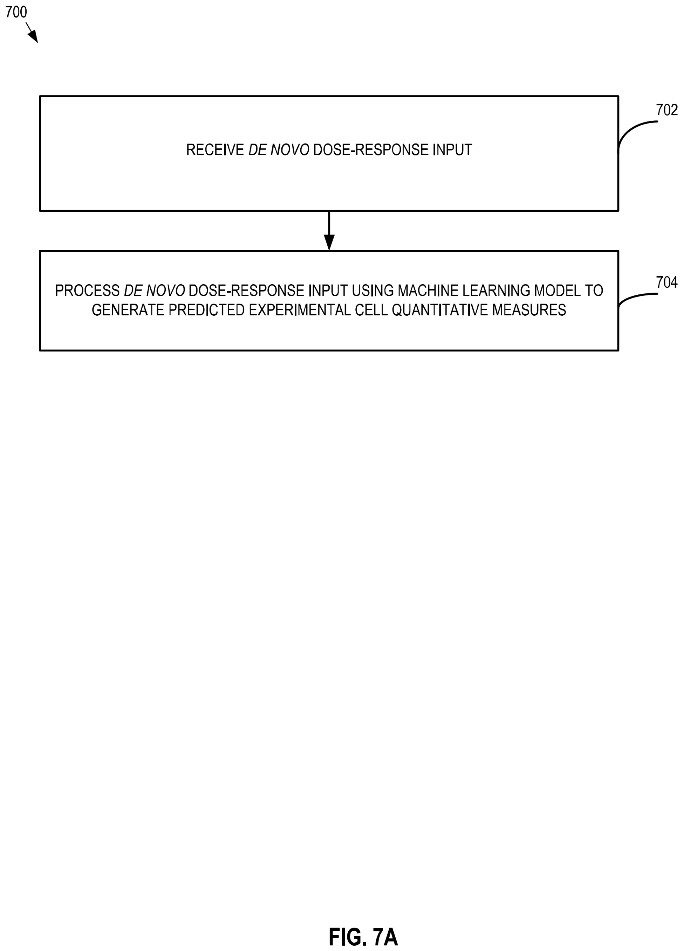

FIG. 7A depicts a computer-implemented method 700 for using a machine learning model trained using quantitative measures data to perform prediction of unmeasured quantitative measures experimental cell quantitative measures to reduce laboratory experimentation burden, according to some aspects. The method 700 may include receiving, via one or more processors, *de novo* quantitative measures input (block 702). The method 700 may include processing the *de novo* quantitative measures input using the machine learning model to generate one or more predicted experimental cell quantitative measures (block 704).

The machine learning model of the method 700 may be a regression machine learning model, a classification machine learning model, etc. The *de novo* quantitative measures input may include a sparse quantitative measures matrix. For example, the *de novo* quantitative measures input may correspond to the incomplete dose matrix block 202 of FIG. 2, and the machine learning model may correspond to the machine learning model block 204 of FIG. 2. In some aspects, the regression machine learning model may be at least one of (i) a random forest model, (ii) an XGBoost model, (iii) an artificial neural network, (iv) a Gaussian regression model, (v) a support vector machine or (vi) a ridge and LASSO regression model.

The regression machine learning model may include a single or multi-target mean-squared-error cost function.

In some aspects, the predicted experimental cell quantitative measures include data corresponding to at least one microscopy image associated with an experiment or experimental results. For example, a data source used to measure viability may be a microscopy fluorescence image. That may be independent of any underlying model used to predict quantitative information. In some aspects, the machine learning model is trained on at least some masked training quantitative measures data.

In some aspects, the machine learning model is an artificial neural network capable of encoding and decoding input images and trained using quantitative measures data including one or more brightfield training images and/or one or more fluorescent training images. In that case, the *de novo* quantitative measures input may correspond to at least one point in a continuous latent space embedding, as discussed for example with respect to FIG. 5D. The artificial neural network may be a variational autoencoder network, a transformer network, a recurrent neural network or a long short-term network. For example, the machine learning model may include an encoder trained to map one or more portions of the brightfield or fluorescent training images to the continuous latent space embedding representing a compressed low dimensional representation of the one or more training images; and a decoder trained to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input. Examples of this architecture are discussed with respect to FIG. 5D, FIG. 5E and FIG. 5F. The method 700 may include training one or more parallel autoencoders, as shown in FIG. 5E.

In some aspects, the method 700 may include predicting viability or synergy scores or other quantitative measures metrics from images using the machine learning model. The method 700 may include augmenting the continuous latent space embedding, using the encoder, by processing one or more human-interpretable morphological features, or other additional features. The human-interpretable morphological features may be generated by a computer-vision technique.

The method 700 may further include generating one or both of (i) a predicted viability corresponding to the *de novo* quantitative measures input, and (ii) a synergy map or score corresponding to the *de novo* quantitative measures input.

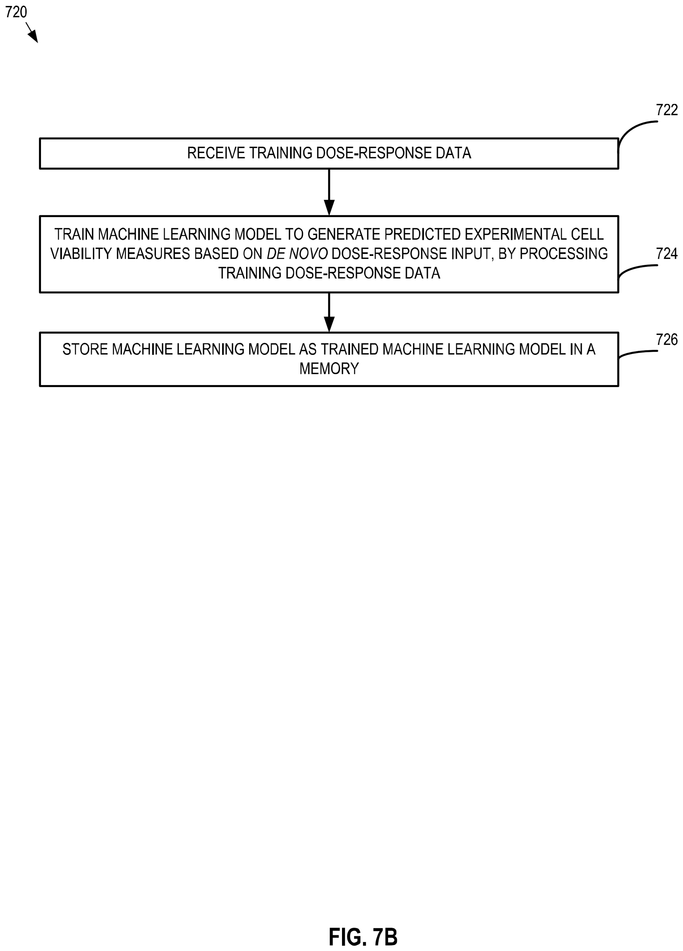

FIG. 7B depicts a computer-implemented method 720 for training a machine learning model to perform prediction of unmeasured quantitative measures quantitative measures to identify compounds showing synergistic effects and reduce laboratory experimentation burden, according to some aspects. The method 720 may include receiving, via one or more processors, training quantitative measures data (block 722). The method 720 may include training, via one or more processors, a machine learning model to generate predicted experimental cell viability measures based on a *de novo* quantitative measures input, by processing the training quantitative measures data (block 724). The method may include storing, via one or more processors, the machine learning model as a trained machine learning model in a memory of a computer (block 726).

The method 720 may be a regression machine learning model. In some aspects, the training quantitative measures data of the method 720 includes one or more substantially dense or fully dense training quantitative measures matrices, each including respective experimental cell viability measures. The *de novo* quantitative measures input may include a sparse quantitative measures matrix. The *de novo* quantitative measures input may correspond to inference-time (i.e., runtime) inputs that the model has not seen previously. The regression machine learning model may be at least one of (i) a random forest model, (ii) an XGBoost model, (iii) an artificial neural network, (iv) a Gaussian regression model, (v) a support vector machine or (vi) a ridge and LASSO regression model. The regression machine learning model may include a single or multi-target mean-squared-error cost function. In some aspects, other cost functions may be used.

The method 720 may include training the machine learning model by receiving, via one or more processors, one or more additional input features; and processing the additional input features along with the one or more substantially dense or fully dense training quantitative measures matrices. For example, the input modality of the additional input features may be binary data, a vector of embeddings, text data, tabular data, etc. The substance of the additional input features may correspond to at least one of (i) a fluorescent nuclear and chromosome counterstain value, (ii) an indication of cell death, (iii) an indication of cell apoptosis, (iv) a cancer type, (v) one or more image embeddings, (vi) one or more morphological features, (vii) one or more compound doses, (viii) one or more features encoding a molecular structure of a compound (ix) RNA sequencing data of cells, or (x) DNA sequencing data of cells. Training the machine learning model may include masking at least some of the training quantitative measures data.

In some aspects, for example as shown in FIGs. 5D-5F, the machine learning model may be a variational autoencoder. The training quantitative measures data may include one or more brightfield and/or fluorescent training images; and the *de novo* quantitative measures input may correspond to at least one point in a continuous latent space embedding. Training the machine learning model may include, via one or more processors, an encoder to map one or more portions of the training images to the continuous latent space embedding representing a compressed low dimensional representation of the one or more training images; and training, via one or more processors, a decoder to map one or more continuous latent space embeddings to one or more portions of a reconstructed image by sampling from the continuous latent space embedding to generate a reconstructed input corresponding to one or more portions of the training images and update, via one or more processors, the continuous latent space embedding based on a comparison between the reconstructed input and the one or more portions of the training input. Training the machine learning model may include training an imaging-based regression model to predict viability or synergy scores from reconstructed images or from the continuous latent space embeddings. The imaging-based regression model may be trained using supervised learning, whereas the autoencoder(s) may be trained using unsupervised learning. Training the imaging-based regression model may include training a fully-connected network to generate decoded viability based on inputs from a decoder.

The method 720 may include receiving human-interpretable morphological features, including at least one of size, shape, circularity, fractal dimension, texture and pixel intensity. The human-interpretable morphological features may be generated by a computer-vision technique, in some aspects. Training the encoder may include augmenting the latent space embedding by processing one or more human-interpretable morphological features. The method 720 may include training a regression head to generate one or both of (i) a predicted viability corresponding to the *de novo* quantitative measures input, and (ii) a synergy map or score corresponding to the *de novo* quantitative measures input. Training the machine learning model may include optimizing the regression head and the variational autoencoder by combining two or more loss functions.

The various embodiments described above can be combined to provide further embodiments. All U.S. patents, U.S. patent application publications, U.S. patent application, foreign patents, foreign patent application and non-patent publications referred to in this specification and/or listed in the Application Data Sheet are incorporated herein by reference, in their entirety. Aspects of the embodiments can be modified if necessary to employ concepts of the various patents, applications, and publications to provide yet further embodiments.

These and other changes can be made to the embodiments in light of the above-detailed description. In general, in the following claims, the terms used should not be construed to limit the claims to the specific embodiments disclosed in the specification and the claims but should be construed to include all possible embodiments along with the full scope of equivalents to which such claims are entitled. Accordingly, the claims are not limited by the disclosure.

## ADDITIONAL CONSIDERATIONS

The computer-readable media may include executable computer-readable code stored thereon for programming a computer (e.g., comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device 1300 may represent a CPU-type processing unit, a GPU-type processing unit, a TPU-type processing unit, a field-programmable gate array (FPGA), another class of digital signal processor (DSP), or other hardware logic components that can be driven by a CPU.

It is noted that while example deep learning frameworks herein have been described as configured with example machine learning architectures (FCN configurations), any number of suitable artificial neural network architectures may be used. Broadly speaking, the deep learning frameworks herein may implement any suitable statistical model (e.g., a neural network or other model implemented through a machine learning process) that will be applied to each of the received images. As discussed herein, that statistical model may be implemented in a variety of manners. In some examples, machine learning is used to evaluate training images.. In some examples, image features can be identified as training classifiers using a learning algorithm such as Neural Network, Support Vector Machine (SVM) or other machine learning process. Once classifiers within the statistical model are adequately trained with a series of training images, the statistical model may be employed in real time to analyze subsequent images provided as input to the statistical model for predicting viability. In some examples, when a statistical model is implemented using a neural network, the neural network may be configured in a variety of ways. In some examples, the neural network may be a deep neural network and/or a convolutional neural network. In some examples, the neural network can be a distributed and scalable neural network. The neural network may be customized in a variety of manners, including providing a specific top layer such as but not limited to a logistic regression top layer. A convolutional neural network can be considered as a neural network that contains sets of nodes with tied parameters. A deep convolutional neural network can be considered as having a stacked structure with a plurality of layers. The neural network or other machine learning processes may include many different sizes, numbers of layers and levels of connectedness. Some layers can correspond to stacked convolutional layers (optionally followed by contrast normalization and max-pooling) followed by one or more fully-connected layers. For neural networks trained by large datasets, the number of layers and layer size can be increased by using dropout to address the potential problem of overfitting. In some instances, a neural network can be designed to forego the use of fully connected upper layers at the top of the network. By forcing the network to go through dimensionality reduction in middle layers, a neural network model can be designed that is quite deep, while dramatically reducing the number of learned parameters.

A system for performing the methods described herein may include a computing device, and more particularly may be implemented on one or more processing units, for example, Central Processing Units (CPUs), and/or on one or more or Graphical Processing Units (GPUs), including clusters of CPUs and/or GPUs. Features and functions described may be stored on and implemented from one or more non-transitory computer-readable media of the computing device. The computer-readable media may include, for example, an operating system and software modules, or "engines," that implement the methods described herein. More generally, the computer-readable media may store batch normalization process instructions for the engines for implementing the techniques herein. The computing device may be a distributed computing system, such as an Amazon Web Services or Google Cloud Platform cloud computing solution.

The computing device includes a network interface communicatively coupled to network, for communicating to and/or from a portable personal computer, smart phone, electronic document, tablet, and/or desktop personal computer, or other computing devices. The computing device further includes an I/O interface connected to devices, such as digital displays, user input devices, etc.

The functions of the engines may be implemented across distributed computing devices, etc. connected to one another through a communication link. In other examples, functionality of the system may be distributed across any number of devices, including the portable personal computer, smart phone, electronic document, tablet, and desktop personal computer devices shown. The computing device may be communicatively coupled to the network and another network. The networks may be public networks such as the Internet, a private network such as that of a research institution or a corporation, or any combination thereof. Networks can include, local area network (LAN), wide area network (WAN), cellular, satellite, or other network infrastructure, whether wireless or wired. The networks can utilize communications protocols, including packet-based and/or datagram-based protocols such as Internet protocol (IP), transmission control protocol (TCP), user datagram protocol (UDP), or other types of protocols. Moreover, the networks can include a number of devices that facilitate network communications and/or form a hardware basis for the networks, such as switches, routers, gateways, access points (such as a wireless access point as shown), firewalls, base stations, repeaters, backbone devices, etc.

The computer-readable media may include executable computer-readable code stored thereon for programming a computer (for example, comprising a processor(s) and GPU(s)) to the techniques herein. Examples of such computer-readable storage media include a hard disk, a CD-ROM, digital versatile disks (DVDs), an optical storage device, a magnetic storage device, a ROM (Read Only Memory), a PROM (Programmable Read Only Memory), an EPROM (Erasable Programmable Read Only Memory), an EEPROM (Electrically Erasable Programmable Read Only Memory) and a Flash memory. More generally, the processing units of the computing device may represent a CPU-type processing unit, a GPU-type processing unit, a field-programmable gate array (FPGA), another class of digital signal processor (DSP), or other hardware logic components that can be driven by a CPU.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components or multiple components.

Additionally, certain aspects are described herein as including logic or a number of routines, subroutines, applications, or instructions. These may constitute either software (e.g., code embodied on a machine-readable medium or in a transmission signal) or hardware. In hardware, the routines, etc., are tangible units capable of performing certain operations and may be configured or arranged in a certain manner. In example aspects, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various aspects, a hardware module may be implemented mechanically or electronically. For example, a hardware module may comprise dedicated circuitry or logic that is permanently configured (e.g., as a special-purpose processor, such as a microcontroller, field programmable gate array (FPGA) or an application-specific integrated circuit (ASIC)) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering aspects in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connects the hardware modules. In aspects in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of the example methods described herein can be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example aspects, comprise processor-implemented modules.

Similarly, the methods or routines described herein may be at least partially processor-implemented. For example, at least some of the operations of a method can be performed by one or more processors or processor-implemented hardware modules. The performance of certain of the operations may be distributed among the one or more processors, not only residing within a single machine, but also deployed across a number of machines. In some example aspects, the processor or processors may be located in a single location (e.g., within a home environment, an office environment or as a server farm), while in other aspects the processors may be distributed across a number of locations.

The performance of certain of the operations may be distributed among the one or more processors, not only residing within a single machine, but also deployed across a number of machines. In some example aspects, the one or more processors or processor-implemented modules may be located in a single geographic location (e.g., within a home environment, an office environment, or a server farm). In other example aspects, the one or more processors or processor-implemented modules may be distributed across a number of geographic locations.

Unless specifically stated otherwise, discussions herein using words such as "processing," "computing," "calculating," "determining," "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one aspect" or "an aspect" means that a particular element, feature, structure, or characteristic described in connection with the aspect is included in at least one aspect. The appearances of the phrase "in one aspect" in various places in the specification are not necessarily all referring to the same aspect.

Some aspects may be described using the expression "coupled" and "connected" along with their derivatives. For example, some aspects may be described using the term "coupled" to indicate that two or more elements are in direct physical or electrical contact. The term "coupled," however, may also mean that two or more elements are not in direct contact with each other, but yet still co-operate or interact with each other. The aspects are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the aspects herein. This is done merely for convenience and to give a general sense of the description. This description, and the claims that follow, should be read to include one or at least one and the singular also includes the plural unless it is obvious that it is meant otherwise.

This detailed description is to be construed as an example only and does not describe every possible aspect, as describing every possible aspect would be impractical, if not impossible. One could implement numerous alternate aspects, using either current technology or technology developed after the filing date of this application.

**Claims**

1.  A computer-implemented method for predicting dose-response quantitative measures by using a machine learning model, the method comprising:

    receiving one or more inputs and one or more additional input features;
    mapping the one or more inputs to a continuous latent space;
    generating at least one first embedding vector in the continuous latent space based on the one or more inputs;
    augmenting the at least one first embedding vector with the one or more additional input features to generate at least one second embedding vector; and
    predicting the dose-response quantitative measures based on the at least one second embedding vector.

2.  The method of claim 1, wherein

    the one or more inputs comprises a brightfield and/or fluorescence image; and/or
    the machine learning model comprises any of: a conventional neural network, a variational autoencoder, a generative adversarial network, a transformer, a **RNN,** a LSTM, an image-based classifier, a fully-connected network, or a regression head; and/or
    the machine learning model is optimized by combining an image reconstruction loss function and a viability prediction loss function; and/or
    the dose-response quantitative measures are represented in any of: predicted cell viability for unmeasured dose combination, predicted cell viability for an unmeasured time point, or predicted synergy map/synergy score of a combination of compounds.

3.  The method of claims 1 or 2, the generating at least one first embedding vector in the continuous latent space based on the one or more inputs comprising:

    identifying more than one treatment points in the continuous latent space based on the one or more inputs;
    interpolating among the more than one treatment points to generate the at least one first embedding vector.

4.  The method of claim 3, wherein the more than one treatment points comprises any of: a point corresponding to an untreated condition, a point corresponding to a first drug, a point corresponding to a second drug, or points acquired at multiple times.

5.  The method of claim 4, the identifying more than one treatment points in the continuous latent space based on the one or more inputs comprising:

    identifying a first treatment point in the continuous latent space based on the one or more inputs, wherein the first treatment point corresponds to a maximum dosage of the first drug; and
    identifying a second treatment point in the continuous latent space based on the one or more inputs, wherein the second treatment point corresponds to a minimum dosage of the first drug.

6.  The method of claim 5, the interpolating between the more than one treatment points to generate the at least one first embedding vector comprising:

    sampling the continuous latent space along a direction of a vector from the second treatment point to the first treatment point to interpolate a third point, wherein the third point corresponds to an unmeasured intermediate dosage of the first drug; and
    generating the at least one first embedding vector based on the third point.

7.  The method of any of claims 3 to 6, after defining at least three treatment points, the interpolating among the more than one treatment points to generate the at least one first embedding vector comprising:

    sampling the continuous latent space along at least two directions of a hyperplane defined by the at least three treatment points to generate data for intermediate conditions; and
    generating the at least one first embedding vector based on the data for intermediate conditions.

8.  The method of any of claims 1 to 7, wherein

the one or more additional input features comprise cell sequencing features and/or drug molecular structure features; or

the one or more additional input features comprise cell sequencing embeddings and/or drug molecular structure embeddings, or

the one or more additional input features comprise morphological human-interpretable features at image level and/or organoid level.

9. The method of claim 8, when the one or more additional input features comprise cell sequencing embeddings and/or drug molecular structure embeddings, the one or more additional input features are obtained by following steps:

receiving one or more features, wherein the one or more features comprise cell sequencing features and/or drug molecular structure features;

mapping the one or more features to the continuous latent space;

generating one or more feature embeddings in the continuous latent space based on the one or more features; and

generating the one or more additional input features based on the one or more feature embeddings.

10. The method of any of claims 1 to 9, the predicting the dose-response quantitative measures based on the at least one second embedding vector comprising:

generating one or more unmeasured point based on the at least one second embedding vector;

generating one or more reconstructed inputs based on the one or more unmeasured points;

predicting the dose-response quantitative measures based on the one or more reconstructed inputs.

11. A computing module for predicting dose-response quantitative measures by using a machine learning model, the module comprising:

a first model configured to

receive one or more inputs and one or more additional input features;

map the one or more inputs to a continuous latent space;

generate at least one first embedding vector in the continuous latent space based on the one or more inputs; and

augment the at least one first embedding vector with the one or more additional input features to generate at least one second embedding vector;

a second model configured to:

predict the dose-response quantitative measures based on the at least one second embedding vector.

12. The module of claim 11, wherein the first model is trained in the following steps:

receiving a training dataset, wherein the training dataset comprises brightfield and/or fluorescence images and/or cell painting images and/or additional features, the training dataset corresponds to a variety of testing conditions; and

reconstructing a training autoencoder output based on the training dataset, wherein the training autoencoder output comprises any of: reconstructed images, reconstructed image embeddings, or reconstructed feature embeddings.

13. The module of claim 12, wherein, after training the first model, the second model is trained in the following steps:

receiving the training autoencoder output from the first model;

providing a label to each of the training autoencoder output; and

predicting endpoints of the training autoencoder output by using the label.

14. A computing system, comprising:

one or more processors; and

one or more memories having stored thereon computer-executable instructions that, when executed by the one or more processors, cause the computing system to perform the method of any of claims 1 to 13.

15. A computer-readable medium having stored thereon computer-executable instructions that, when executed by one or more processors, cause a computer to perform the method of one of claims 1 to 13.

100

QUANTITATIVE MEASURES
PREDICTION COMPUTING DEVICE
102

PROCESSORS
120

NIC
122

MEMORY
124

MODULES 130

QUANTITATIVE
MEASURES DATA
152

MACHINE LEARNING
TRAINING
154

MACHINE LEARNING
OPERATION
156

VISUALIZATION
158

QUANTITATIVE MEASURES
CLIENT COMPUTING DEVICE
104

VIEWER APPLICATION
170

NETWORK
106

QUANTITATIVE
MEASURES DATA
GENERATION SYSTEM
108

110

INPUT
DEVICE
126

OUTPUT
DEVICE
128

FIG. 1

FIG. 2

FIG. 3

400

402

404

FIG. 4A

450

FIG. 4B

500

502-A

**Input**

504-A

502-A

**x**

506-A

Probabilistic
Encoder

$[\mu, \sigma]$

**Sampled latent
vector**

**Z**

506-B

Probabilistic
Decoder $p_{\ominus}$
$(x|z)$

502-B

**x'**

502-B

**Reconstructed
input**

504-B

**FIG. 5A**

FIG. 5B

FIG. 5C

FIG. 5D

**FIG. 5E**

FIG. 5F

EP 4 664 464 A2

**FIG. 6**

700

┌─────────────────────────────────────────────────────────────────┐
│                                                                   │ 702
│              RECEIVE *DE NOVO* DOSE-RESPONSE INPUT                 │
│                                                                   │
└─────────────────────────────────────────────────────────────────┘
                                  │
                                  ▼
┌─────────────────────────────────────────────────────────────────┐
│   PROCESS *DE NOVO* DOSE-RESPONSE INPUT USING MACHINE LEARNING MODEL TO  │ 704
│   GENERATE PREDICTED EXPERIMENTAL CELL QUANTITATIVE MEASURES       │
│                                                                   │
└─────────────────────────────────────────────────────────────────┘

**FIG. 7A**

720

```
                                                              722
┌─────────────────────────────────────────────────┐
│        RECEIVE TRAINING DOSE-RESPONSE DATA        │
└─────────────────────────────────────────────────┘
                        │
                        ▼
                                                              724
┌─────────────────────────────────────────────────┐
│ TRAIN MACHINE LEARNING MODEL TO GENERATE PREDICTED│
│ EXPERIMENTAL CELL VIABILITY MEASURES BASED ON     │
│ DE NOVO DOSE-RESPONSE INPUT, BY PROCESSING        │
│ TRAINING DOSE-RESPONSE DATA                       │
└─────────────────────────────────────────────────┘
                        │
                        ▼
                                                              726
┌─────────────────────────────────────────────────┐
│ STORE MACHINE LEARNING MODEL AS TRAINED MACHINE   │
│ LEARNING MODEL IN A MEMORY                        │
└─────────────────────────────────────────────────┘
```

**FIG. 7B**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 81639522 A
- US 816395
- US 30197521 A

- US 85790122 A
- US 11438620 A
- US 301975

**Non-patent literature cited in the description**

- **LANEVSKI ; ALEKSANDR ; ANIL K. GIRI ; TERO AITTOKALLIO**. SynergyFinder 3.0: an interactive analysis and consensus interpretation of multi-drug synergies across multiple samples. *Nucleic acids research*, 2022, vol. 50 (W1), W739-W743

- **WOOTEN, DAVID J ; RÉKA ALBERT.** synergy: a Python library for calculating, analyzing and visualizing drug combination synergy. *Bioinformatics*, 2021, vol. 37 (10), 1473-1474